(19) Europäisches Patentamt
European Patent Office
Office européen des brevets

(11) EP 2 521 554 B1

(12) **EUROPEAN PATENT SPECIFICATION**

(45) Date of publication and mention of the grant of the patent:
**22.11.2017 Bulletin 2017/47**

(21) Application number: **10840657.0**

(22) Date of filing: **30.12.2010**

(51) Int Cl.:
*A61K 31/7048 (2006.01)* *A61K 31/7032 (2006.01)*
*A61K 31/215 (2006.01)* *A61K 31/192 (2006.01)*
*A61K 36/899 (2006.01)* *A61K 36/634 (2006.01)*
*A61K 36/538 (2006.01)* *A61K 36/534 (2006.01)*
*A61K 36/484 (2006.01)* *A61K 36/48 (2006.01)*
*A61K 36/35 (2006.01)* *A61K 36/34 (2006.01)*
*A61K 36/28 (2006.01)* *A61P 31/16 (2006.01)*

(86) International application number:
**PCT/IB2010/003482**

(87) International publication number:
**WO 2011/080593 (07.07.2011 Gazette 2011/27)**

(54) **MATERIALS AND METHODS FOR PREVENTION AND TREATMENT OF VIRAL INFECTIONS**

MATERIALIEN UND VERFAHREN ZUR VORBEUGUNG UND BEHANDLUNG VON VIRUSINFEKTIONEN

SUBSTANCES ET PROCÉDÉS POUR PRÉVENTION ET TRAITEMENT D'INFECTIONS VIRALES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.12.2009 US 291073 P**

(43) Date of publication of application:
**14.11.2012 Bulletin 2012/46**

(73) Proprietors:
- **BAGI Research Limited**
  **1 Connaught Place Central**
  **Hong Kong (CN)**
- **Versitech Limited**
  **Hong Kong (CN)**

(72) Inventors:
- **LAU, Allan, Sik-Yin**
  **Hong Kong (CN)**
- **YANG, Lai, Hung, Cindy**
  **Hong Kong (CN)**
- **LAW, Anna, Hing-Yee**
  **Hong Kong (CN)**

(74) Representative: **Engelhard, Markus**
**Boehmert & Boehmert**
**Anwaltspartnerschaft mbB**
**Pettenkoferstrasse 22**
**80336 München (DE)**

(56) References cited:
**WO-A1-99/34812** **CN-A- 1 192 357**
**CN-A- 1 348 813** **CN-A- 1 504 232**
**CN-A- 101 342 185** **CN-A- 101 380 330**
**CN-A- 101 390 869** **CN-A- 101 390 869**
**US-A- 4 078 145** **US-A- 6 013 632**
**US-A- 6 063 383**

- **DATABASE CA [Online] CHEMICAL ABSTRACTS SERVICE, COLUMBUS, OHIO, US; LI, HONG: "A pharmaceutical composition having antibacterial and antiviral effects", XP002699493, retrieved from STN Database accession no. 2009:1357671 & CN 1 154 246 A (PEOP. REP. CHINA) 16 July 1997 (1997-07-16)**

EP 2 521 554 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **DATABASE MEDLINE [Online] US NATIONAL LIBRARY OF MEDICINE (NLM), BETHESDA, MD, US; August 2009 (2009-08), WANG XING ET AL: "[Determination of forsythoside A in fructus forsythiae by NIR].", XP002699494, Database accession no. NLM19938549 & WANG XING ET AL: "[Determination of forsythoside A in fructus forsythiae by NIR].", ZHONGGUO ZHONG YAO ZA ZHI = ZHONGGUO ZHONGYAO ZAZHI = CHINA JOURNAL OF CHINESE MATERIA MEDICA AUG 2009, vol. 34, no. 16, August 2009 (2009-08), pages 2071-2075, ISSN: 1001-5302**
- **SU, MEIYING ET AL.: 'Pharmacokinetics of Caffeic Acid in Rats.' CHINA PHARMACY vol. 19, no. 16, 2008, page 1220, XP008163518**

## Description

### BACKGROUND OF THE INVENTION

Viral infections are responsible for many acute and chronic life-threatening diseases. It is estimated that about 33.4 million people are living with human immunodeficiency virus (HIV) worldwide $_{(1)}$. In addition, an estimated 2 billion people have been infected with hepatitis B virus, and 600,000 people die each year due to the acute or chronic consequences of the infection. $_{(2)}$.

Influenza is an acute and one of the most widely spread viral infections worldwide. Major influenza A pandemics include the Asian flu pandemic in 1957 (H2N2), the Hong Kong flu pandemic in 1968 (H3N2), the re-emergence of H1N1 (Russian flu) in 1970, the H5N1 bird flu in 1997 and 2003, and the most recent outbreak of the swine flu (H1N1) in April 2009. As of November 2009, worldwide more than 207 countries and territories or communities have reported laboratory confirmed cases of pandemic influenza H1N1 2009, including at least 8768 deaths $_{(3)}$. In addition, the avian influenza H5N1 virus still presents a significant health concern. According to the World Health Organization, H5N1 results in a high mortality rate of more than 60% as per reported cases.

Despite extensive efforts, the development of effective anti-viral drugs has largely been empirical. There is no cure for HIV. Also, there is no specific treatment for acute hepatitis, although anti-viral treatments may improve the conditions of some patients with chronic hepatitis infection. In addition, almost all circulating strains of seasonal influenza A viruses are resistant to FDA approved antiviral drugs, including the adamantanes and neuraminidase inhibitors $_{(4,5)}$.

CN 101390869 A discloses forsythoside A having an anti-viral effect and inhibiting the growth of influenza A.

US 6,013,632 discloses pharmaceutical compositions comprising caffeic acid and its use for preventing or treating influenza virus infection.

Meiying et al, Pharmacokinetics of Caffeic Acid in Rats, China Pharmacy, 2008, vol. 19, no. 16, p. 1220 discloses the use of caffeic acid for the treatment of viral infections.

US 4,078,145 discloses the use of jacaranone for the treatment of viral infections.

US 6,063,383 discloses pharmaceutical suppository composites for fever and influenza.

In previous research the current inventors have identified a series of potent inhibitors of the H5N1, H1N1 and H9N2 neuraminidases at a level comparable to the known neuraminidase inhibitor oseltamivir $_{(6)}$. They have also used bioactivity-guided purification technologies to analyze and identify the bioactive molecules responsible for anti-inflammatrory actvities in medicinal herbs including Panax ginseng and Cimicifuga species $_{(7,8)}$. While these advances are impressive, efforts to isolate and identify useful therapeutic compounds from natural sources remain difficult and largely empirical. Also, as virus strains change over time, the emergence of resistant mutations further diminishes the efficacy of anti-viral agents. Therefore, the development of additional novel anti-viral therapeutics is urgently needed.

### BRIEF SUMMARY OF THE INVENTION

The present invention relates to an isolated compound being forsythoside A (compound A1)

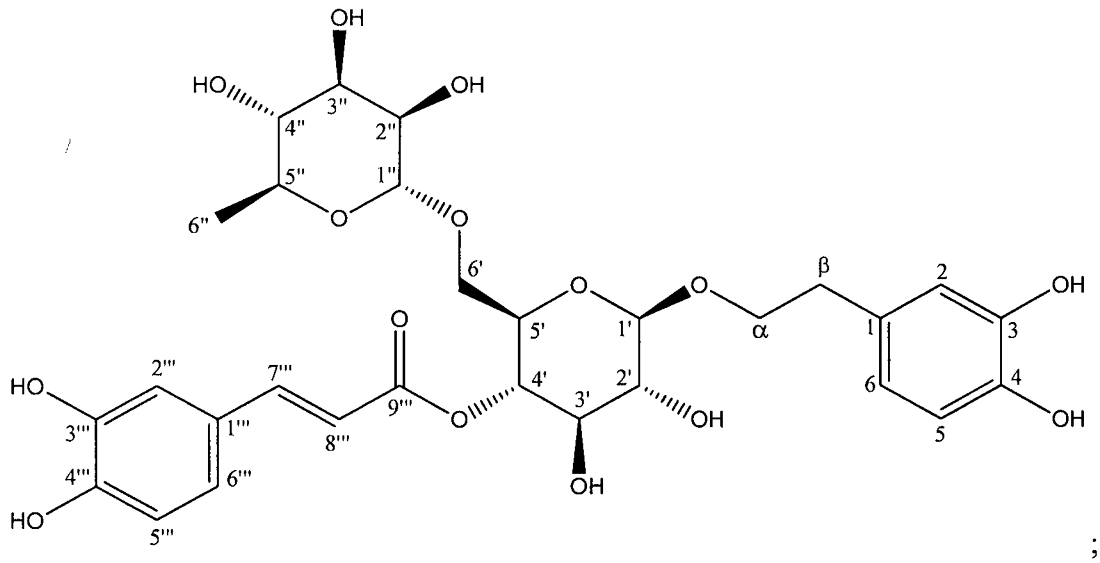

;

or a salt thereof; for use in a method for preventing and/or treating viral infection caused by influenza virus in a subject,

wherein said method comprises administering, to a subject in need of such prevention and/or treatment, an effective amount of said compound, wherein said method is used to prevent and/or treat an infection caused by an oseltamivir-resistant H1N1 influenza virus.

Moreover, it also relates to a therapeutic composition comprising an antiviral amount of an isolated compound , said compound being forsythoside A (compound A1)

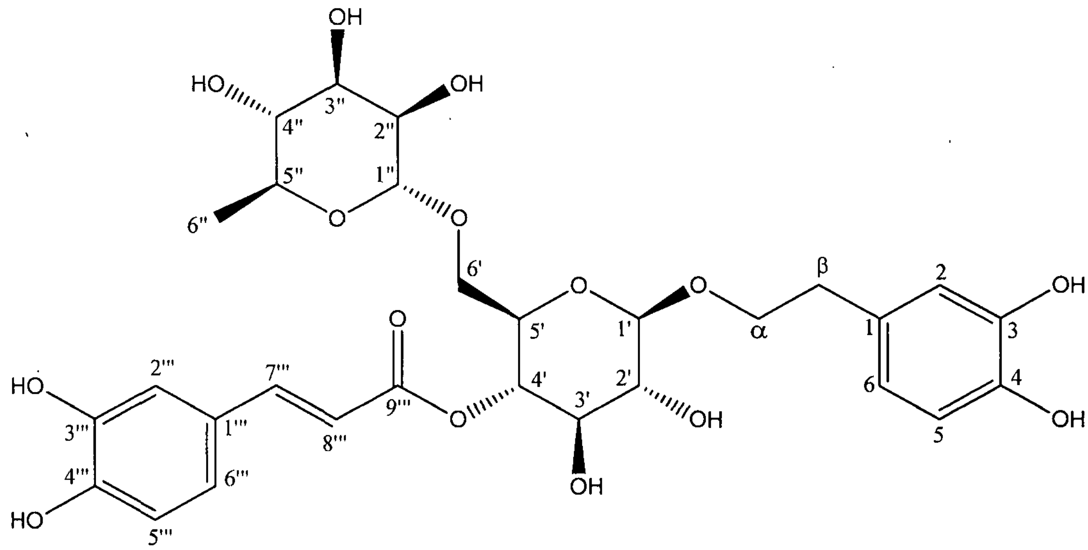

;

or a salt thereof;

for use in a method for preventing and/or treating viral infection caused by influenza virus in a subject, wherein said method comprises administering, to a subject in need of such prevention and/or treatment an effective amount of said compound, wherein said method is used to prevent and/or treat an infection caused by an oseltamivir-resistant H1N1 influenza virus.

The present invention provides novel and advantageous materials and methods for preventing and/or treating viral infection. Specifically exemplified herein are therapeutic uses of forsythoside A and jacaranone, compounds isolated from traditional Chinese medicinal material such as *Fructus forsythiae* (Lian Qiao). Also provided are herbal formulations, and compositions comprising the herbal formulation, for preventing and/or treating viral infection. Preferably, the compounds and compositions of the present invention are formulated for oral administration.

The present invention also provides use of forsythoside A or salts thereof, as medicaments for the prevention and/or treatment of viral infections in accordance with the appended claims.

In a further embodiment, the method comprises administering a composition comprising isolated compounds in a form, including but not limited to, a salt, stereoisomer, tautomer, crystalline, polymorph, amorphous, solvate, hydrate, ester, prodrug, metabolite, and any combination thereof.

Also disclosed are therapeutic methods for treating, preventing, or ameliorating a viral infection by administering an effective amount of the Yin Qiao San (YQS) composition, or a fraction thereof, to a subject. Also provided is the use of the Yin Qiao San composition, or a fraction thereof, as medicament for treating, preventing, or ameliorating viral infection.

Also disclosed are fractions of the Yin Qiao San composition, fractionated by reversed-phase HPLC method described herein. Disclosed are one or more fractions F2 - F5 of a methanol extract of the Yin Qiao San composition, whose HPLC chromatograph is shown in Figure 12. Also disclosed are fraction F2 and/or F3 of the methanol extract of the Yin Qiao San composition, whose HPLC chromatograph is shown in Figure 12. Also disclosed is fraction S11 of the Yin Qiao San composition, whose HPLC chromatograph is shown in Figure 14.

In one embodiment, the disclosed methods can be used to prevent and/or treat viral infection caused by influenza virus A, B or C, including but not limited to, H1N1, H9N2, H3N2, H5N1, H2N2, H7N7, H7N1, and any combination thereof.

In a further embodiment, the methods for preventing and/or treating viral infection further comprise: administering to a subject a second anti-viral agent selected from, for example, zanamivir, oseltamivir, peramivir, seltamivir, and any of the combination thereof; and wherein the second antiviral agent can be administered prior to, simultaneously with, or subsequent to the administration of a compound of the subject invention.

In a still further embodiment, the methods for preventing and/or treating viral infection further comprise administering to a subject, in addition to a compound of the present invention, a traditional Chinese medicinal material including, but not limited to, *Fructus forsythiae, Herba seu Flos Schizonepetae Tenuifoliae, Fructus Arctii Lappae, Semen Sojae*

*Preparatum, Herba Lophatheri Gracilis, Radix Glycyrrhizae Uralensis,* and *Radix Platycodi Grandiflori.*

In a still further embodiment, the methods for treating viral infection further comprise a step of determining the level of viral infection in a subject; wherein the determination is optionally made at multiple times to monitor the change over time.

In another further embodiment, the methods for treating viral infection further comprise a step of determining the level of one or more bio-markers in a subject; wherein the determination is optionally made at multiple times to monitor the change over time.

BRIEF DESCRIPTION OF THE FIGURES

**Figure 1** shows an extraction scheme of bioactive compounds including forsythoside A (Compound A1) and jacaranone (Compound D1) from *Fructus forsythiae. Fructus forsythiae* is milled and extracted with 10x of milli-Q water under reflux and boiled for 2 hours. The extraction process is repeated once. Each time, the resulting extract is collected, combined, evaporated to dryness, and re-dissolved in MeOH. Using a bioassay guided isolation scheme, the fractions showing significant anti-viral and/or COX-2 suppressive effects are further purified using the reversed-phase high-performance liquid chromatography (HPLC) until pure compounds are obtained.

**Figures 2A-B** show reverse-phase high performance liquid chromatography (HPLC) chromatograms **(A)** and UV absorbance **(B)** of forsythoside A (Compound A1). Compound A1 is purified by reversed-phase HPLC using gradient elution from 10% to 90% of acetonitrile at a flow rate of 1 mL/min. **Figure 2A** shows that a single peak is detected using Photo-diode Array detector at 254, 210 and 280 nm. Compound A1 is eluted at approximate 6.8 min. **Figure 2B** shows that the UV absorbance of Compounc A1 is maximized at 210, 290 and 330 nm.

**Figures 3A-B** show reverse-phase high performance liquid chromatography (HPLC) chromatograms **(A)** and UV absorbance **(B)** of jacaranone (Compound D1). Compound D1 is purified by reversed-phase HPLC using gradient elution from 10% to 90% of acetonitrile at a flow rate of 1 mL/min. **Figure 3A** shows that a single peak is detected using Photo-diode Array detector at 254, 210 and 280 nm. Compound D1 is eluted at approximately 10.75 min. **Figure 3B** shows that UV absorbance of Compound D1 is maximized at 230 nm.

**Figure 4** shows the $^1H$ (upper panel) and $^{13}C$ (lower panel) chemical shifts of forsythoside A (Compound A1). The structure of Compound A1 is elucidated by a Bruker 500 MHz DRX NMR spectrometer, operating at 500 MHz for $^1H$ and at 125 MHz for $^{13}C$ NMR, using methanol-*d* with TMS as the solvent.

**Figure 5** shows the $^1H$ (upper panel) and $^{13}C$ (lower panel) chemical shifts of jacaranone (Compound D1). The structure of Compound D1 is elucidated by a Bruker 500 MHz DRX NMR spectrometer, operating at 500 MHz for $^1H$ and at 125 MHz for $^{13}C$ NMR, using methanol-*d* with TMS as the solvent.

**Figure 6** shows the chemical structures of forsythoside A and jacaranone.

**Figures 7A-E** show inhibitory effects of forsythoside A (Compound A1) or oseltamivir on influenza virus replication. MDCK cells are infected with influenza viruses **(A)** H1N1 (A/HK/54/98), **(B)** H1N1-R (A/Vicotria/07159200/07), **(C)** H9N2 (A/Quail/HK/G1/97) or **(D)** H3N2 (A/H3N2/1174/99). After 48-hour incubation with forsythoside A (Compound A1) (100ug/ml), viral titers ($TCID_{50}$) in cell culture supernatant are measured by titration in MDCK cells. **(E)** After infecting the cells with H1N1 (A/HK/54/98) virus, oseltamivir (100 $\mu$M) or PBS are added to the cells as controls. Viral titers ($TCID_{50}$) in cell culture supernatant are measured by titration in MDCK cells. (All figures are representative results from three experiments).

**Figure 8** shows inhibitory effects of forsythoside A (Compound A1) on influenza virus replication in primary human blood macrophages. Primary human blood macrophages are infected with human influenza viruses including H1N1 (A/HK/54/98); H9N2 (A/Quail/HK/G1/97); and H3N2 (A/H3N2/1174/99) at an m.o.i. of 2. Cells are then incubated with forsythoside A (Compound A1) (100 $\mu$g/ml) or DMSO for 48 hours. After that, cell culture supernatants are collected and viral titers ($TCID_{50}$) are measured by titration in MDCK cells. (Representative results from three experiments.)

**Figures 9A-C** show inhibitory effects of forsythoside A (Compound A1) on influenza virus as determined by immunocytochemistry **(A-B)** and Western blot analysis **(C)**. **(A)** MDCK cells are infected with human influenza virus H9N2/G1 (A/Quail/HK/G1/97) at an m.o.i. of 0.2. Cells are then incubated with Compound A1 (100$\mu$g/ml) or DMSO for 6 or 10 hours. After that, cells are fixed. Influenza nucleoprotein and M1 protein are stained with the Influenza Detection Kit. **(B)** The numbers of cells expressing viral proteins at 6 and 10 hour post-infection are counted and percentages of cells expressing viral proteins are calculated. **(C)** MDCK cells are infected with human influenza virus H9N2/G1 (A/Quail/HK/G1/97) at an m.o.i. of 2 or mock infected (M). After that, cells are then incubated with forsythoside A (Compound A1) (100$\mu$g/ml) or DMSO. Proteins are harvested at 2, 4, 9 or 24 hour post-infection. Expressions of the influenza M1 protein are analyzed by Western blot analysis. Actin is used as a loading control. A & C, representative figures from three experiments; B, n=3, *$P$<0.05.

**Figures 10A-D** show the ultrastructural examination of virions by transmission electron microscopy. MDCK cells are infected with human influenza virus H9N2/G1 (A/Quail/HK/G1/97) at an m.o.i. of 2. Cells are then incubated with forsythoside A (Compound A1) (100μg/ml) **(C-D)** or DMSO **(A-B)** for 18 hours. Cells are then fixed and ultrastructures are examined by transmission electron microscopy. Arrows in (B) and (D) indicate influenza virions. Magnifications: A, 3200×; B, 6300×; C, 4800×; D, 8900×.

**Figures 11A-B** show inhibitory effects of jacaranone (Compound D1) on cyclooxygenase 2 (COX-2). **(A)** Primary human blood macrophages are infected with H9N2/G1 (A/Quail/HK/G1/97) at an m.o.i. of 2. Cells are then incubated with Compound D1 (10μg/ml or 20μg/ml) or DMSO for 3 hours. After that, total RNA are harvested and COX-2 mRNA levels are examined by TaqMan Gene Expression assays (n=5). **(B)** Cells are either infected with H9N2/G1 (AQuail/HK/G1/97) at an m.o.i. of 2 or mock-infected. After that, cells are treated with Compound D1 (10μg/ml or 20μg/ml) for 48 hours. Cells culture supernatants are then harvested and prostaglandin $E_2$ levels are measured by the Prostaglandin $E_2$ EIA Kit (n=4). **P*<0.05; #*P*<0.01; ##*P*<0.005.

**Figure 12** shows reverse-phase high performance liquid chromatography (HPLC) chromatogram of Yin Qiao San (YQS) formula. The fractions are separated by reversed-phase HPLC using gradient elution from 10% to 90% of acetonitrile at a flow rate of 1 mL/min. Total five fractions are obtained.

**Figures 13A-C** show inhibitory effect of YQS and YQS fractions on virus replications. MDCK cells are infected with H1N1 (A/HK/54/98) **(A)** or H9N2/G1 (A/Quail/HK/G1/97) **(B)** at an m.o.i. of 2. Cells are then incubated with YQS (100μg/ml) or DMSO for 48 hours. After that, cell culture supernatants are collected and viral titers ($TCID_{50}$) are measured by titration in MDCK cells. (C) MDCK cells are infected with H9N2/G1 (A/Quail/HK/G1/97) at an m.o.i. of 2. Cells are then incubated with YQS fractions (F1 - F5) (100μg/ml) or DMSO for 48 hours. After that, cell culture supernatants are collected and viral titers ($TCID_{50}$) are measured by titration in MDCK cells. Representative figures from three **(A-B)** or two **(C)** independent experiments.

**Figure 14** shows a typical chromatogram of YQS obtained by reverse-phase high performance liquid chromatography (HPLC). The fractions are separated by reversed-phase HPLC using gradient elution from 10% to 90% of acetonitrile at a flow rate of 1 mL/min. Total thirteen fractions are obtained.

**Figures 15A-B** show YQS and YQS fractions induce interleukin (IL)-1β. **(A)** Primary human blood macrophages are infected with H1N1 (A/HK/54/98) at an m.o.i. of 2 or mock infected. H1N1-infected cells are then incubated with YQS (100μg/ml) or DMSO for 3 hours. After that, total RNA are harvested and IL-1β mRNA levels are examined by TaqMan Gene Expression assays. **(B)** Primary human blood macrophages are infected with H1N1 (A/HK/54/98) at an m.o.i. of 2 or mock infected. H1N1-infected cells are then incubated with YQS fractions (S1 - S13) (100μg/ml) or DMSO for 3 hours. After that, total RNA are harvested and IL-1β mRNA levels are examined by TaqMan Gene Expression assays. Representative figures from three experiments.

DETAILED DESCRIPTION OF THE INVENTION

The subject invention provides novel and advantageous materials and methods for preventing, treating and/or ameliorating viral infection in a subject, in accordance with the appended claims, namely therapeutic uses of forsythoside A, a compound isolated from traditional Chinese medicinal material such as *Fructus forsythiae* (Lian Qiao). Also provided are herbal formulations, and compositions thereof, for preventing and/or treating viral infection in accordance with the appended claims. Preferably, the compounds and compositions of the present invention are formulated for oral administration.

The present invention provides use of forsythoside A or salts thereof, as medicaments for the prevention and/or treatment of viral infections in accordance with the appended claims.

"Alkyl" means linear saturated monovalent radicals of one to eight carbon atoms or a branched saturated monovalent of three to eight carbon atoms. It may include hydrocarbon radicals of one to four or one to three carbon atoms, which may be linear. Examples include methyl, ethyl, propyl, 2-propyl, n-butyl, iso-butyl, tert-butyl, pentyl, and the like.

"Acyl" means a radical -C(O)R where R is, for example, hydrogen, alkyl or cycloalkyl, heterocycloalkyl, halo, or alkyl halo. Examples further include formyl, acetyl, ethylcarbonyl, and the like.

"Carboxyl" means the radical -C(O)OH.

"Carboalkoxy" means a radical -C(O)R where R is, for example, hydrogen, alkyl or cycloalkyl, heterocycloalkyl, halo, or alkyl halo.

"Carboxamide" means a radical -C(O)R, where R is, for example, $-NH_2$ or Alkylamino.

"Halo" means fluoro, chloro, bromo, or iodo, such as bromo and chloro.

"Haloalkyl" means alkyl substituted with one or more, same or different, halo atoms, e.g., $-CH_2Cl$, $-CH_2Br$, $-CF_3$, $-CH_2CH_2Cl$, $-CH_2CCl_3$, and the like.

"Amino" means the radical $-NH_2$.

"Alkylamino" means a radical -NHR or $NR_2$ where each R is, independently, an alkyl group. Examples include

methylamino, (1-methylethyl)amino, dimethylamino, methylethylamino, di(1-methylethyl)amino, and the like.

"Hydroxy" means the radical -OH.

"Hydroxyalkyl" means an alkyl radical as defined herein, substituted with one or more, preferably one, two or three, hydroxy groups. Representative examples include, but are not limited to, hydroxymethyl, 2-hydroxyethyl, 2-hydroxypropyl, 3-hydroxypropyl, 1-(hydroxymethyl)-2-methylpropyl, 2-hydroxybutyl, 3-hydroxybutyl, 4-hydroxybutyl, 2,3-dihydroxypropyl, 2-hydroxy-1-hydroxymethylethyl, 2,3-dihydroxybutyl, 3,4-dihydroxybutyl and 2- (hydroxymethyl)-3-hydroxy-propyl, preferably 2-hydroxyethyl, 2,3- dihydroxypropyl and 1- (hydroxymethyl) 2-hydroxyethyl.

"Alkoxy" means the radical -$OR_a$, where $R_a$ is an alkyl group. Exemplary alkoxy groups include methoxy, ethoxy, propoxy, and the like.

Also disclosed are methods for preventing, treating, or ameliorating viral infections by administering a composition comprising isolated enantiomeric compounds. The isolated enantiomeric forms of the compounds of the invention are substantially free from one another (i.e., in enantiomeric excess). In other words, the "R" forms of the compounds are substantially free from the "S" forms of the compounds and are, thus, in enantiomeric excess of the "S" forms. Conversely, "S" forms of the compounds are substantially free of "R" forms of the compounds and are, thus, in enantiomeric excess of the "R" forms. In one embodiment of the invention, the isolated enantiomeric compounds are at least about in 80% enantiomeric excess. In a preferred embodiment, the compounds are in at least about 90% enantiomeric excess. In a more preferred embodiment, the compounds are in at least about 95% enantiomeric excess. In an even more preferred embodiment, the compounds are in at least about 97.5% enantiomeric excess. In a most preferred embodiment, the compounds are in at least about 99% enantiomeric excess.

Also disclosed are methods for preventing, treating, or ameliorating viral infections by administering a composition comprising isolated compounds in a form, including but not limited to, a salt, stereoisomer, tautomer, crystalline, polymorph, amorphous, solvate, hydrate, ester, prodrug, metabolite, and any combination thereof.

The term "prodrug," as used herein, refers to a metabolic precursor of a compound of the present invention or pharmaceutically acceptable form thereof. In general, a prodrug comprises a functional derivative of a compound, which may be inactive when administered to a subject, but is readily convertible *in vivo* into an active metabolite compound.

Conventional procedures for the selection and preparation of suitable prodrug derivatives are described, for example, in "Design of Prodrugs", ed. H. Bundgaard, Elsevier, 1985. Preferably, a prodrug of the present invention enhances desirable qualities of the compound of the present invention, including not limited to, solubility, bioavailability, and stability. Hence, the compounds employed in the present methods may, if desired, be delivered in a prodrug form. Prodrugs of the compounds employed in the present invention may be prepared by modifying functional groups present in the compound such that the modifications are cleaved, either in routine manipulation or *in vivo,* to the parent compound.

The term "metabolite," refers to a pharmacologically active product, including for example, an active intermediate or an ultimate product, produced through *in vivo* metabolism of a compound of the present invention in a subject. A metabolite may result, for example, from the anabolic and/or catabolic processes of the administered compound in a subject, including but not limited to, the oxidation, reduction, hydrolysis, amidation, deamidation, esterification, deesterification, enzymatic cleavage, and the like.

Metabolites are typically identified by preparing a radiolabeled (e.g., $^{14}C$ or .$^{3}H$) isotope of a compound of the present invention, administering it parenterally in a detectable dose (e.g., greater than about 0.5 mg/kg) to an animal such as rat, mouse, guinea pig, monkey, or to a human, allowing sufficient time for metabolism to occur (typically about 30 seconds to about 30 hours), and isolating its conversion products from the urine, blood or other biological samples. These products are easily isolated since they are labeled (others are isolated by the use of antibodies capable of binding epitopes surviving in the metabolite). The structure of metabolites can be determined in conventional fashion, e.g., by MS, LC/MS or NMR analysis. In general, analysis of metabolites is performed according to techiniques well known to those skilled in the art of drug metabolism studies.

The term "subject," as used herein, describes an organism, including mammals such as primates, to which treatment with the compositions according to the present invention can be provided. Mammalian species that can benefit from the disclosed methods of treatment include, but are not limited to, apes, chimpanzees, orangutans, humans, monkeys; and domesticated animals such as dogs, cats, horses, cattle, pigs, sheep, goats, chickens, mice, rats, guinea pigs, and hamsters.

The term "antiviral," as used herein, includes but is not limited to, preventing, inhibiting, suppressing, reducing, adversely impacting, and/or interfering with the growth, survival, replication, function, and/or dissemination of a virus.

Also disclosed are therapeutic methods for treating, preventing, or ameliorating viral infection by administering an effective amount of the compounds of the subject invention.

The subject invention provides for forsythoside A for use in therapeutic methods for treating, preventing, ameliorating viral infection by administering, an effective amount of forsythoside A (Compound A1) or salt thereof, to a subject. The chemical structure of forsythoside A (Compound A1) is:

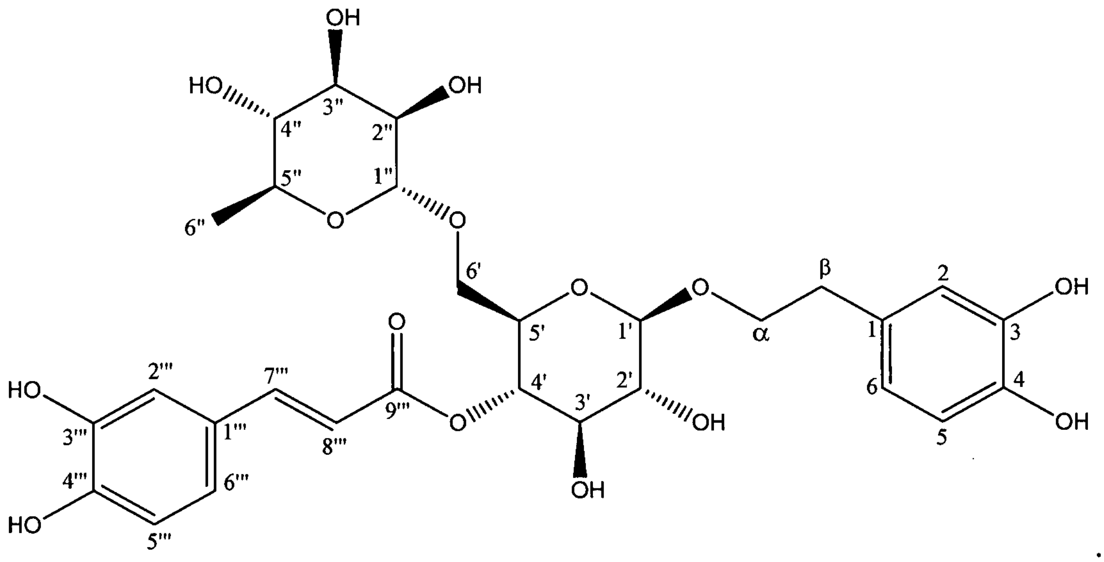

Also disclosed are therapeutic methods for treating, preventing, and/or ameliorating viral infection by administering, an effective amount of jacaranone (Compound D1) or a prodrug, metabolite or salt thereof, to a subject. The chemical structure of jacaranone (Compound D1) is:

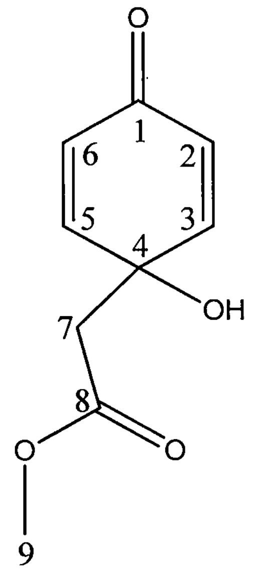

Forsythoside A (Compound A1) and jacaranone (Compound D1) can be isolated from *Fructus forsythiae* and related plants using isolation and bioassay-guided procedures as described herein.

Also disclosed are therapeutic methods for treating, preventing, or ameliorating viral infection by administering, an effective amount of prodrug and/or metabolite of Compound A1 (or salt thereof), to a subject. An exemplified metabolite of Compound A1 is Compound B1. The chemical structure of Compound B1 is:

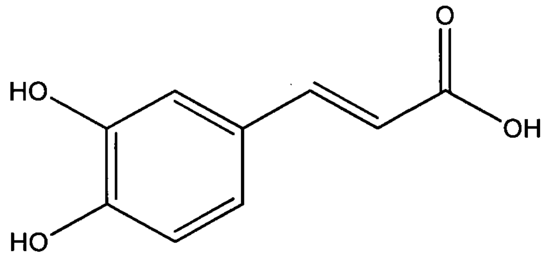

Another exemplified metabolite of Compound A1 is Compound C1. The chemical structure of Compound C1 is:

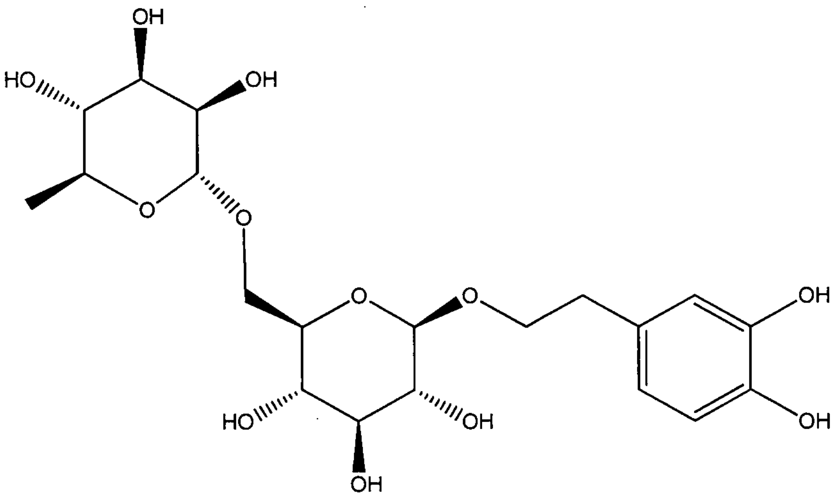

Also disclosed are therapeutic methods for treating, preventing, or ameliorating viral infection by administering an effective amount of any of a combination of forsythoside A (Compound A1), Compound B1, Compound C1, and Compound D1 (and prodrugs, metabolites or salts thereof) to a subject.

Also disclosed are herbal formulations, and compositions comprising the herbal formulation (improved Yin Qiao San formulations), for preventing and/or treating viral infection.

In one embodiment, the composition comprises an herbal formulation consisting of herbs having relative weight amounts as indicated in the parenthesis: Lian Qiao / *Fructus forsythiae suspensae* (25%-35%), Jin Yin Hua / *Flos Lonicerae Japonicae* (25%-35%), Niu Bang Zi / *Fructus Arctii Lappae* (1 %-7% or 15%-25%), Jie Geng / *Radix Platycodi Grandiflori* (1%-7%), Bo He / *Herba menthae haplocalycis* (1%-7%), Dan Dou Chi / *Semen Sojae Preparatum* (3%-7%), Gan Cao / *Radix Glycyrrhizae Uralensis* (8%-20%), Dan Zhu Ye / *Herba Lophatheri Gracilis* (1%-5%), and Jing Jie (1%-5%).

Also disclosed is a herbal formulation consisting of herbs having relative weight amounts as indicated in the parenthesis: Lian Qiao / *Fructus forsythiae suspensae* (25%-40%), Jin Yin Hua / *Flos Lonicerae Japonica* (25%-40%), Niu Bang Zi / *Fructus Arctii Lappae* (15%-25%), and Gan Cao / *Radix Glycyrrhizae Uralensis* (10%-20%); and wherein the composition does not comprise any of the following herbs: Jing Jie, Bo He / *Herba menthae haplocalycis*; Dan Dou Chi / *Semen Sojae Preparatum,* Dan Zhu Ye / *Herba Lophatheri Gracilis,* and Jie Geng / *Radix Platycodi Grandiflori.*

Also disclosed is a composition comprising the herbal formulation of YQS-F9 as shown in Table 1. In certain preferred embodiments, the composition comprises an herbal formulation selected from YQS-F5, YQS-F6A, or YQS-F1 as shown in Table 1. In certain embodiments, the composition comprises one or more herbal formulations as shown in Table 1. In an embodiment, the composition does not comprise the YQS-Traditional formulation as shown in Table 1.

Table 1 illustrates embodiments of the herbal formulations of the subject invention.

**Table 1: Percentage Distribution of herbal constituents in Ying Qiao San (YQS).**

| Herbs | YQS-F1 | YQS-F2 | YQS-F3 | YQS-F4 | YQS-F5 | YQS-F6A | YQS-F9 Preferred | YQS-Traditional |
|---|---|---|---|---|---|---|---|---|
| Lianqiao (FFS) | 32% | 33% | 23.5% | 22.2% | 30.8% | 30% | 30% | 22% |
| Jin yin hua (FLJ) | 32% | 17% | 23.5% | 22.2% | 30.8% | 20% | 30% | 22% |

(continued)

| Herbs | YQS-F1 | YQS-F2 | YQS-F3 | YQS-F4 | YQS-F5 | YQS-F6A | YQS-F9 Preferred | YQS-Traditional |
|---|---|---|---|---|---|---|---|---|
| Niu bang zi (FAL) | 19% | 33% | 23.5% | 22.2% | 15.4% | 20% | 5% | 9% |
| Jie geng (RPG) | - | - | 1.4% | 2.7% | 1.8% | 4% | 5% | 9% |
| Bo he (HMH) | - | - | 1.4% | 2.7% | 1.8% | 4% | 5% | 9% |
| Dan dou chi (SSP) | - | - | 1.2% | 2.2% | 1.5% | 4% | 5% | 7.5% |
| Gancao (RGU) | 16% | 17% | 23.5% | 22.2% | 15.4% | 10% | 10% | 7.5% |
| Dan zhu ye (HLG) | - | - | 0.9% | 1.8% | 1.2% | 4% | 5% | 6% |
| Jing jie (HST) | - | - | 0.9% | 1.8% | 1.2% | 4% | 5% | 6% |
| **Anti-viral effects (% of suppression)** | 85% | <50% | <50% | ~50% | 80% | 85% | ~80% | |

Total Dry Weight in Traditional YQS Decoction is 66 g.

Advantageously, the compositions of the subject invention provide surprisingly superior anti-viral effects. Specifically, the compositions of the subject invention enhance IL-1β level in a subject who has viral infection.

In an embodiment, the subject invention provides therapeutic methods for treating, preventing, or ameliorating viral infection by administering an effective amount of the composition of the subject invention, or a fraction thereof, to a subject. Also provided is use of the composition of the subject invention, or a fraction thereof, as a medicament for treating, preventing, or ameliorating viral infection in accordance with the appended claims.

Also disclosed are therapeutic methods for treating, preventing, or ameliorating viral infection by administering an effective amount of the Yin Qiao San (YQS) composition (YQS-traditional formulation as shown in Table 1), or a fraction thereof, to a subject. Also provided is use of the Yin Qiao San composition, or a fraction thereof, as a medicament for treating, preventing, or ameliorating viral infection.

The Yin Qiao San composition is an herbal mixture consisting of *Herba menthae haplocalycis, Herba seu Flos Schizonepetae Tenuifoliae, Fructus forsythiae suspensae, Fructus Arctii Lappae, Semen Sojae Preparatum, Herba Lophatheri Gracilis, Radix Glycyrrhizae Uralensis, Flos Lonicerae Japonicae,* and *Radix Platycodi Grandiflori.*

The Yin Qiao San composition consists of herbs having relative weight amounts as indicated in the parenthesis: *Herba menthae haplocalycis* (about 60), *Herba seu Flos Schizonepetae Tenuifoliae* (about 40), *Fructus forsythiae suspensae* (about 100), *Fructus Arctii Lappae* (about 60), *Semen Sojae Preparatum* (about 50), *Herba Lophatheri Gracilis* (about 40), *Radix Glycyrrhizae Uralensis* (about 50), *Flos Lonicerae Japonicae* (about 100) and *Radix Platycodi Grandiflori* (about 60).

Also disclosed are fractions of the Yin Qiao San composition, fractionated by reversed-phase HPLC (Lichrospher 100 RP C18 EC 5 μm, 250×4.6 mm ID) using a gradient elution from 10% acetonitrile ($CH_3CN$) to 90% $CH_3CN$ at a flow rate of 1 mL/min. Peak detection is achieved using an Agilent 1200 series of fast scanning Photo-diode Array detector set at 210, 254 and 280 nm.

Also disclosed are one or more fractions F2 - F5 of the Yin Qiao San composition, whose HPLC chromatograph is shown in Figure 12. Also disclosed is fraction F2 and/or F3 of the Yin Qiao San composition, whose HPLC chromatograph is shown in Figure 12. Also disclosed is fraction S11 of the Yin Qiao San composition, whose HPLC chromatograph is shown in Figure 14.

The term "treating," as used herein, includes but is not limited to, reducing, suppressing, inhibiting, lessening, or affecting the progression, severity, and/or scope of a condition, chance of re-occurrence or returning of a disease after a remission. In one embodiment, treating may include directly affecting or curing, suppressing, inhibiting, reducing the severity of, delaying the onset of, reducing symptoms associated with an infection, or a combination thereof. In another embodiment, treating includes delaying progression, expediting remission, inducing remission, augmenting remission, speeding recovery, increasing efficacy of or decreasing resistance to alternative therapeutics, or a combination thereof.

The term "suppressing" or "inhibiting," as used herein, includes but is not limited to, reducing the severity of symptoms, reducing the severity of an acute episode, reducing the number of symptoms, reducing the incidence of disease-related symptoms, reducing the latency of symptoms, ameliorating symptoms, reducing secondary symptoms, reducing secondary infections, prolonging patient survival, or a combination thereof.

The term "preventing," as used herein, includes but is not limited to, delaying the onset of symptoms, preventing relapse to a disease, decreasing the number or frequency of relapse episodes, increasing latency between symptomatic

episodes, or a combination thereof.

The term "effective amount," as used herein, refers to an amount that is capable of preventing, ameliorating, or treating viral infection. The term "effective amount" also includes an amount that is capable of preventing, ameliorating, or treating viral infections and/or inhibiting or reducing the level of COX-2 and/or PGE2. In certain embodiments, the effective amount enables at least 5%, 10%, 15%, 20%, 25%, 30%, 35%, 40%, 45%, 50%, 60%, 70%, 80%, 90%, or 100% decrease in viral titers (*e.g.*, $TCID_{50}$). The decrease in viral titers can be determined from, for example, biological samples obtained from a subject at different time points.

Advantageously, the compounds and compositions of the subject invention are capable of preventing, inhibiting, reducing and/or retarding viral replication. In specific embodiments, the replication of influenza viruses H1N1 (A/HK/54/98), H1N1 (A/Vicotria/07159200/07), H9N2 (A/Quail/HK/G1/97) and H3N2 (A/H3N2/1174/99) in MDCK cells is suppressed by forsythoside A (Compound A1) for about 15-, 3-, 24- and 12-fold, respectively.

In addition, the compounds and compositions of the subject invention are useful for modulating immune responses during viral infection. For instance, the compounds and compositions of the subject invention enhance IL-1β levels. The compounds and compositions of the subject invention can also inhibit COX-2 and prostaglandin $E_2$ (PGE-2) activity, particularly during viral infection. According to the present invention, the novel and advantageous therapeutic method is capable of preventing, treating, or ameliorating viral infection of a subject resistant to existing anti-viral therapies, including but not limited to, compounds such as adamantanes and neuraminidase inhibitors, zanamivir, oseltamivir, peramivir and seltamivir, interferons, nucleosides, siRNAs, or any of the combination thereof.

The compounds, pharmaceutical compositions, and therapeutic methods of the subject invention are useful for preventing, treating, or ameliorating infections caused by influenza viruses, including but not limited to: any of the subtypes of influenza A, influenza B, or influenza C.

The term "influenza virus," as used herein, includes any strain of influenza viruses that is capable of causing disease in an animal or human subject, or that is an interesting candidate for experimental analysis. Influenza viruses are described in Fields, B., et al., Fields' Virology, 4th ed., Philadelphia: Lippincott Williams and Wilkins; ISBN: 0781718325, 2001.

Influenza A virus, as used herein, encompasses any strain of influenza A virus that is capable of causing disease in an animal or human subject, or that is an interesting candidate for experimental analysis. A large number of influenza A isolates have been partially or completely sequenced, as is described in *see* Macken, C., Lu, H., Goodman, J., & Boykin, L., "The value of a database in surveillance and vaccine selection." in Options for the Control of Influenza IV. A.D.M.E. Osterhaus, N. Cox & A. W. Hampson (Eds.) Amsterdam: Elsevier Science, 2001, 103-106). This database also contains complete sequences for influenza B and C genome segments.

Influenza viruses are enveloped, negative-stranded RNA viruses of the Orthomyxoviridae family. They are classified as influenza types A, B, and C, of which influenza A is the most pathogenic and is believed to be the only type able to undergo re-assortment with animal strains. Influenza types A, B, and C can be distinguished by differences in their nucleoprotein and matrix proteins. As discussed further below, influenza A subtypes are defined by variation in their hemagglutinin (HA) and neuraminidase (NA) genes and usually distinguished by antibodies that bind to the corresponding proteins.

The influenza A viral genome consists of ten genes distributed in eight RNA segments. The genes encode 10 proteins: the envelope glycoproteins hemagglutinin (HA) and neuraminidase (NA); matrix protein (M1); nucleoprotein (NP); three polymerases (PB1, PB2, and PA) which are components of an RNA-dependent RNA transcriptase also referred to as a polymerase or polymerase complex herein; ion channel protein (M2), and nonstructural proteins (NS1 and NS2). The organization of the influenza B viral genome is extremely similar to that of influenza A whereas the influenza C viral genome contains seven RNA segments and lacks the NA gene.

Influenza A virus classification is based on the hemagglutinin and neuraminidase genes. Potential hemagglutinin and neuraminidase genes can be determined using the UniProtKB/Swiss-Prot Release 57.5 (07-Jul-2009) or the UniProtKB/TrEMBL Release 40.5 (07-Jul-2009), as is known in the art. World Health Organization (WHO) nomenclature defines each virus strain by its animal host of origin (specified unless human), geographical origin, strain number, year of isolation, and antigenic description of HA and NA. For example, human epidemics have been caused by viruses with HA types H1, H2, and H3 and NA types N1 and N2. For example, the H5N1/97 "bird-flu" incident was the first documented direct transmission of an avian influenza virus to humans, causing devastating infections with severe viral pneumonia and a mortality rate of > 30% $_{(9)}$. Other influenza A epidemics include the Asian flu pandemic in 1957 (H2N2), the Hong Kong flu pandemic in 1968 (H3N2), the re-emergence of H1N1 (Russian flu) in 1970, and the most recent swine flu H1N1 in April 2009.

In one embodiment, the subject compounds, pharmaceutical compositions, and therapeutic methods are useful for preventing, treating, or ameliorating infections caused by influenza A viruses, including but not limited to, any of the strains of H1N1, H1N2, H1N3, H1N4, H1N5, H1N6, H1N7, H1N8, H1N9, H2N1, H2N2, H2N3, H2N4, H2N5, H2N6, H2N7, H2N8, H2N9, H3N1, H3N2, H3N3, H3N4, H3N5, H3N6, H3N7, H3N8, H3N9, H4N1, H5N2, H5N3, H5N4, H5N5, H5N6, H5N7, H5N8, H5N9, H6N1, H6N2, H6N3, H6N4, H6N5, H6N6, H6N7, H6N8, H6N9, H7N1, H7N2, H7N3, H7N4,

H7N5, H7N6, H7N7, H7N8, H7N9, H8N1, H8N2, H8N3, H8N4, H8N5, H8N6, H8N7, H8N8, H8N9, H9N1, H9N2, H9N3, H9N4, H9N5, H9N6, H9N7, H9N8, H9N9, H10N1, H10N2, H10N3, H10N4, H10N5, H10N6, H10N7, H10N8, H10N9, H11N1, H11N2, H11N3, H11N4, H11N5, H11N6, H11N7, H11N8, H11N9, H12N1, H12N2, H12N3, H12N4, H12N5, H12N6, H12N7, H12N8, H12N9, H13N1, H13N2, H13N3, H13N4, H13N5, H13N6, H13N7, H13N8, H13N9, H14N1, H14N2, H14N3, H14N4, H14N5, H14N6, H14N7, H14N8, H14N9, H15N1, H15N2, H15N3, H15N4, H15N5, H15N6, H1N7, H15N8, and H15N9.

In a specific embodiment, the subject compounds, pharmaceutical compositions, and therapeutic methods are useful for preventing, treating, or ameliorating infections caused by influenza A viruses, including but not limited to, any of the strains of H1N1, H9N2, H3N2, H5N1, H2N2, H7N7, and H7N1.

In another specific embodiment, the subject compounds, pharmaceutical compositions, and therapeutic methods are useful for preventing, treating, or ameliorating infections caused by influenza A viruses, including but not limited to, H1N1 (A/HK/54/98, oseltamivir-sensitive strain), H1N1 (A/Vicotria/07159200/07, oseltamivir-resistant strain), H9N2 (A/Quail/HK/G1/97), H3N2 (A/H3N2/1174/99), H5N1(A/H5N1/97), H1N1 (A/54/98), H9N2/G1, and H6N1 (A/Teal/HK/W312/97).

As is known in the art, genetic variation occurs by two primary mechanisms in viruses such as influenza A. Genetic drift occurs via point mutations, which often occur at antigenically significant positions due to selective pressure from host immune responses, and genetic shift, involving substitution of a whole viral genome segment of one subtype by another. In addition, many different types of animal species including humans, swine, birds, horses, aquatic mammals, and others, may become infected with viruses. Therapeutic methods useful for preventing, treating, or ameliorating of infections caused by viral variants are embodiments of the subject invention.

In addition, the subject compounds, pharmaceutical compositions, and therapeutic methods are useful for preventing, treating, or ameliorating infections caused by pathogens, including but not limited to, bacteria, fungi, parasitic microorganisms, RNA, DNA, retroviral vectors, tumor/oncogenic viruses, pirons, protozoan, environmental toxins, and a combination of infectious pathogens.

The presence and/or level of a type of virus or multiple types of viruses can be determined from a sample of biological fluid obtained for the purpose of evaluation of a subject of interest, such as a patient. The presence and/or level of the viruses can be measured in a biological sample such as, blood, tissue, serum, plasma, urine, saliva, and tears. In one embodiment, the sample is a blood sample. In another embodiment, the sample is a urine sample. In another embodiment, the sample is a saliva sample. In another embodiment, the sample is a bodily fluid sample.

The presence and/or level of a type of virus or multiple types of viruses can be determined in a manner as is known in the art, such as for example a titration, a viral titer, enzyme-linked immunosorbant assay (ELISA), western blot, and immunological assays.

Upon viral infection, drastic changes are rapidly induced in the host cells. Among various consequences caused by viral infections, a shared feature is the shifting of the cellular response to pathogens from apoptotic death to autophagy $_{(10)}$. The switch to the autophagy state is associated with the induction of pro-inflammatory cytokines in the virus-infected human macrophages $_{(10)}$. For instance, coronavirus and poliovirus are capable of inducing the autophagy processes in the host cells in favor of the survival and replication of viruses.

Influenza virus-infected human macrophages exhibit delayed onset of apoptosis and hyper-induction of a variety of pro-inflammatory cytokines and related molecules. For instance, the H5N1/97 virus is found to induce high levels of pro-inflammatory cytokines in differentiated primary human blood macrophages $_{(11)}$. This cytokine dysregulation contributes to the pathogenesis and severity of the disease $_{(12-13)}$. In addition, p38K, a mitogen-activated protein kinase (MAPK), also plays a significant role in the hyper-induction of TNF-$\alpha$ in H5N1-infected macrophages $_{(14)}$. Influenza such as H5N1 and H9N2/G1 infection also triggers the hyper-induction of IFN-$\beta$ and IFN-$\alpha$ (including IFN-$\alpha$ subtypes such as IFNA1, 2 and 8) in human blood macrophages. Additionally, interferon regulatory factor 3 (IRF3) plays a significant role in the hyper-induction of the cytokines including IFN-$\beta$, IFN-$\lambda$1 and TNF-$\alpha$ in human macrophages infected with H5N1 viruses $_{(15)}$.

A switch of cellular response from apoptotic death to the autophagy state, associated with the induction of pro-inflammatory cytokines in the host cells, is also found in other pathogenic infections such as bacterial, fungal, and microbial infections, including infections caused by intracellular microbes such as Listeria and Mycobacteria.

The compounds of the subject application have useful immunomodulatory properties by regulating or assisting the regulation of cytokines and cytokine-related molecules during infection. Also, these cytokines and related molecules can serve as bio-markers, such as for the determination of the severity of the condition and progression of the infection, appropriateness of therapeutic methods, dosage, route of administration, and/or the need for administration of other pharmaceutical agents.

The biomarkers include, but are not limited to, TNF-$\alpha$; Interleukin-1beta (IL-1$\beta$), COX-2, prostaglandin (*e.g.*, PEG2), interferons such as Interferon-beta (IFN-$\beta$), Interferon-alpha (IFN-$\alpha$) which includes IFN-$\alpha$ subtypes such as IFNA1, 2 and 8, and Interferon-gamma (IFN-$\gamma$); p38K; IRF3; the interleukin family such as Interleukin-1 (IL-1), Interleukin-2 (IL-2), Interleukin-3 (IL-3), Interleukin-4 (IL-4), Interleukin-5 (IL-5), Interleukin-6 (IL-6), Interleukin-7 (IL-7), Interleukin-8 (IL-8), Interleukin-9 (IL-9), Interleukin-10 (IL-10), Interleukin-11 (IL-11), Interleukin-12 (IL-12), Interleukin-13 (IL-13), Interleukin-14 (IL-14), Interleukin-15 (IL-15), Interleukin-16 (IL-16), Interleukin-17 (IL-17), Interleukin-18 (IL-18), Interleukin-19 (IL-19), Interleukin-20 (IL-20), Interleukin-21 (IL-21), Interleukin-22 (IL-22), Interleukin-23 (IL-23), Interleukin-24 (IL-24), Interleukin-25 (IL-25), Interleukin-26 (IL-26), Interleukin-27 (IL-27), Interleukin-28 (IL-28), Interleukin-29 (IL-29), Interleukin-30 (IL-30), Interleukin-31 (IL-31), Interleukin-32 (IL-32), Interleukin-33 (IL-33), Interleukin-34 (IL-34), Interleukin-35 (IL-35); the interleukin receptor family; the macrophage inflammatory protein family such as macrophage inflammatory protein 2 (MIP-2) and macrophage inflammatory protein 1$\alpha$ (MIP-1 $\alpha$); macrophage colony-stimulating factor (M-CSF); monocyte chemotactic protein-1 (MCP-1); and immunoglobulins such as IgA, IgG, IgM, IgD, and IgE.

Immunoglobulins include IgG, IgM, IgD, IgE, IgA and subtypes such as for example IgG1, IgG2, IgG3, IgG4, IgA1, and IgA2. They further include molecules in monomeric or multimeric form, whether digested from whole antibody or produced by other means.

In a further specific embodiment, cytokines and related molecules modulated by the compounds of the subject invention are selected from the group consisting of TNF-$\alpha$, IFN-$\beta$, IFN-$\alpha$ including subtypes 1, 2 and 8, IFN-$\gamma$, p38K, IRF3, IL-1, IL-2, IL-3, IL-5, IL-4, IL-6, IL-8, IL-10, IL-12, IL-13, IL-14, IL-17, IL-18, IL-23, IL-24, IL-25, IL27, IL-32, G-CSF, M-CSF, MCP-1, MIP-2, MIP-1$\alpha$, IgA, IgG, IgM, IgD and IgE.

The presence and/or level of the bio-markers can be determined from a sample of biological fluid obtained from a subject of interest, such as a patient. The presence and/or level of the viruses can be measured in a sample such as, blood, tissue, serum, plasma, urine, saliva, and tears. In one embodiment, the sample is a blood sample. In another embodiment, the sample is a urine sample. In another embodiment, the sample is a saliva sample. In another embodiment, the sample is a bodily fluid sample.

In a further embodiment, the level of the bio-marker can be determined by quantitative immunological detection methods, such as for example enzyme-linked immunosorbant assay (ELISA), western blot, immunological assays, microarray and radioimmunoassay.

Further, a plurality of markers can be measured. In addition, analysis of a plurality of markers may be carried out separately or simultaneously. Several markers may be combined into one test for efficient processing of multiple samples from a subject.

In one embodiment, the subject invention provides a therapeutic method by administering isolated compounds. As used herein, "isolated" refers to compounds that have been removed from any environment in which they may exist in nature. For example, isolated forsythoside A would not refer to the forsythoside A compound as it exists in *Fructus forsythiae.* In preferred embodiments, the compounds of the subject invention are at least 75% pure, preferably at least 90% pure, more preferably are more than 95% pure, and most preferably are more than 99% pure (substantially pure).

In a further specific embodiment, a therapeutic composition of the subject invention includes compounds A1 (forsythoside A), or salts thereof, and any combination thereof, in the absence of other forsythoside compounds.

The present invention also provides for a therapeutic method by administering therapeutic or pharmaceutical compositions in a form that can be combined with a pharmaceutically acceptable carrier. In this context, the compound may be, for example, isolated or substantially pure. The term "carrier" refers to a diluent, adjuvant, excipient, or vehicle with which the compound is administered. Such pharmaceutical carriers can be sterile liquids, such as water and oils, including those of petroleum oil such as mineral oil, vegetable oil such as peanut oil, soybean oil, and sesame oil, animal oil, or oil of synthetic origin. Saline solutions and aqueous dextrose and glycerol solutions can also be employed as liquid carriers, particularly for injectable solutions.

Suitable methods of administration include, but are not limited to, oral, inhalation, or parenteral administration including intravenous, subcutaneous, topical, transdermal, intradermal, transmucosal, intraperitoneal, intramuscular, intracapsular, intraorbital, intracardiac, transtracheal, subcutaneous, subcuticular, intraarticular, subcapsular, subarachnoid, intraspinal, epidural and intrasternal injection, infusion, and electroporation, as well as co-administration as a component of any medical device or object to be inserted (temporarily or permanently) into a subject.

In one embodiment, the subject method further comprises administering to a subject a second anti-viral agent, including but not limited to, compounds such as adamantanes and neuraminidase inhibitors, zanamivir, oseltamivir, peramivir, and seltamivir; interferons; nucleotides; siRNAs; or any of the combination thereof; and wherein the second antiviral agent can be administered prior to, subsequent to, or simultaneous with the compounds or pharmaceutical compositions of the subject invention.

In a specific embodiment, the subject method further comprises administering to a subject a second anti-viral agent, selected from the group consisting of zanamivir, oseltamivir, peramivir, seltamivir, and any of the combination thereof; wherein the second antiviral agent can be administered prior to, subsequent to, or simultaneous with the compounds or pharmaceutical compositions of the subject invention.

In another specific embodiment, the subject compounds or pharmaceutical compositions can be used in combination with at least one second anti-viral agent, optionally administered together with at least one second anti-viral agent as a vaccine; or administered prior to, simultaneously with, or subsequent to at least one second anti-viral agent.

Suitable pharmaceutical excipients include starch, glucose, lactose, sucrose, gelatin, malt, rice, flour, chalk,

silica gel, sodium stearate, glycerol monostearate, talc, sodium chloride, dried skim milk, glycerol, propylene, glycol, water, ethanol and the like. The therapeutic composition, if desired, can also contain minor amounts of wetting or emulsifying agents, or pH buffering agents. These compositions can take the form of solutions, suspensions, emulsion, tablets, capsules, powders, sustained-release formulations and the like. The composition can be formulated with traditional binders and carriers such as triglycerides. Examples of suitable pharmaceutical carriers are described in "Remington's Pharmaceutical Sciences" by E. W. Martin. Such compositions contain a therapeutically effective amount of the therapeutic composition, together with a suitable amount of carrier so as to provide the form for proper administration to the patient. The formulation should suit the mode of administration.

In one embodiment, the composition is formulated in accordance with routine procedures as a pharmaceutical composition adapted for local injection administration to human beings. Typically, compositions for local injection administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is administered by injection, an ampoule of sterile water for injection or saline can be provided so that the ingredients may be mixed prior to administration.

The therapeutic or pharmaceutical compositions of the invention can be formulated as neutral or salt forms. Pharmaceutically acceptable salts include, but are not limited to, hydrochloric, phosphoric, acetic, oxalic, tartaric acids, sodium, potassium, ammonium, calcium, ferric hydroxides, isopropylamine, triethylamine, 2-ethylamino ethanol, histidine, procaine, etc.

The present invention also provides for the modification of the compound such that it is more stable once administered to a subject, i.e., once administered it has a longer time period of effectiveness as compared to the unmodified compound. Such modifications are well known to those of skill in the art, e.g., microencapsulation, etc.

The amount of the therapeutic or pharmaceutical composition of the invention which is effective in the treatment of a particular disease, condition or disorder will depend on the nature of the disease, condition or disorder and can be determined by standard clinical techniques. In general, the dosage ranges from about 0.001 mg/kg to about 2 mg/kg. In addition, in vitro assays may optionally be employed to help identify optimal dosage ranges. The precise dose to be employed in the formulation will also depend on the route of administration, and the seriousness of the disease, condition or disorder, and should be decided according to the judgment of the practitioner and each patient's circumstances. Effective doses may be extrapolated from dose-response curves derived from *in vitro* or animal model test systems. For example, in order to obtain an effective mg/kg dose for humans based on data generated from rat studies, the effective mg/kg dosage in rats is divided by six.

The invention also provides a pharmaceutical pack or kit comprising one or more containers filled with one or more of the ingredients, e.g., compound, carrier suitable for administration.

The method of administration can also be practiced consistent with traditional Chinese medicine practices. The composition and dosage of the formulation that are effective in the treatment of a particular disease, condition or disorder will depend on the nature of the disease, condition or disorder by standard clinical techniques.

The traditional Chinese medicine in prescription amounts can be readily made into any form of drug, suitable for administering to humans or animals. Suitable forms include, for example, tinctures, decoctions, and dry extracts. These can be taken orally, applied through venous injection mucous membranes or inhalation. The active ingredient can also be formulated into capsules, powder, pallets, granules, tablets, pastille, suppositories, oral solutions, pasteurized gastroenteric suspension injections, small or large amounts of injection, frozen powder injections, pasteurized powder injections and the like. All of the above-mentioned methods are known to people skilled in the art, described in books and commonly used by practitioners of herbal medicine.

A tincture is prepared by suspending herbs in a solution of alcohol, such as, for example, wine or liquor. After a period of suspension, the liquid (the alcohol solution) may be administered for example, two or three times a day, one teaspoon each time.

A decoction is a common form of herbal preparation. It is traditionally prepared in a clay pot, but can also be prepared in glass, enamel or stainless steel containers. The formulation can be soaked for a period of time in water and then brought to a boil and simmered until the amount of water is reduced by, for example, half.

An extract is a concentrated preparation of the essential constituents of a medicinal herb. Typically, the essential constituents are extracted from the herbs by suspending the herbs in an appropriate choice of solvent, typically, water, ethanol/water mixture, methanol, butanol, iso-butanol, acetone, hexane, petroleum ether or other organic solvents.

The extracting process may be further facilitated by means of maceration, percolation, repercolation, counter-current extraction, turbo-extraction, or by carbon-dioxide hypercritical (temperature/pressure) extraction. After filtration to rid of herb debris, the extracting solution may be further evaporated and thus concentrated to yield a soft extract (extractum spissum) and/or eventually a dried extract (extractum siccum), by means of spray drying, vacuum oven drying, fluid-bed drying or freeze-drying. The soft extract or dried extract may be further dissolved in a suitable liquid to

a desired concentration for administering or processed into a form such as pills, capsules, injections, etc.

In one embodiment, the subject method further comprises administering to a subject, in addition to a compound of the present invention, a traditional Chinese medicinal material, including but not limited to, *fructus forsythiae, Herba seu Flos Schizonepetae Tenuifoliae, Fructus Arctii Lappae, Semen Sojae Preparatum, Herba Lophatheri Gracilis, Radix Glycyrrhizae Uralensis,* and *Radix Platycodi Grandiflori.*

Materials and Methods

*Plant Material*

The medicinal herbs, *Herba menthae haplocalycis* (60g), *Herba seu Flos Schizonepetae Tenuifoliae* (40g), *Fructus forsythiae suspensae* (100g), *Fructus Arctii Lappae* (60g), *Semen Sojae Preparatum* (50g), *Herba Lophatheri Gracilis* (40g), *Radix Glycyrrhizae Uralensis* (50g), *Flos Lonicerae Japonicae* (100g) and *Radix Platycodi Grandiflori* (60g) as well as herbal extracts are provided by PuraPharm International (H.K.) Ltd.

*Extraction and Isolation of Bioactive Molecules*

*Fructus forsythiae* obtained from Purapharm International (H.K.) Ltd. is dried and grounded into powders. Methods for extracting bioactive compounds are shown in Figure 1 and illustrated as follows.

Briefly, *Fructus forsythiae* (100g) is dried, grounded into powders, and then extracted twice for bioactive compounds. During each extraction, powders are suspended in 10x

milli-Q water under reflux for 2 hours. The supernatant from each extraction is collected, combined and evaporated to dryness under vacuum to yield 22.23g light yellowish powders. The powders are re-dissolved in MeOH and fractionated.

The resulting MeOH extract is purified by reversed-phase high-performance liquid chromatography (HPLC) (Lichrospher 100 RP C18 EC 5 $\mu$m, 250×4.6 mm ID) using a gradient elution from 10% acetonitrile ($CH_3CN$) to 90% $CH_3CN$ at a flow rate of 1 mL/min.

Peak detection is achieved using an Agilent 1200 series of fast scanning Photo-diode Array detector set at 210, 254 and 280 nm. Eluting peaks are scanned between 200 nm and 300 nm with 1 nm intervals to determine absorbance maxima and minima. A total of 13 fractions are obtained.

Each fraction is examined for its biological activities. Fraction 4 shows COX-2 inhibitory effects, and fraction 5 exhibits significant anti-viral effects; thus, they are subject to further purification. By repeating the purification process using HPLC, a pure compound (Compound A1) (6.1mg) with anti-viral effects and a pure compound (Compound D1) (2.8mg) with COX-2 inhibitory effects are obtained.

*Elucidation of the Molecular Structure*

The structure of the pure compounds is elucidated through $^1H$ and $^{13}C$ NMR spectrometry as well as through TOF-ESI-MS and EI-MS spectrometry. The NMR spectra are recorded on a Bruker 500 MHz DRX NMR spectrometer, operating at 500 MHz for $^1H$ and at 125 MHz for $^{13}C$ NMR, using methanol-*d* with TMS as the solvent. The accurate mass determination for Compound A1 is recorded on a micrOTOF II ESI-TOF mass spectrometer (Bruker Daltonics), and the sample is dissolved in MeOH. The accurate mass determination for Compound D1 is recorded on a 5975C EI-MS (Agilent Technologies).

*Preparation of Yingqiaosan (YQS) extract*

The YQS extract is prepared according to the Chinese Pharmacopeia (2005). Briefly, 3g of *Herba menthae haplocalycis* and 2g of *Herba seu Flos Schizonepetae Tenuifoliae* are extracted twice with 20-fold milli-Q water under reflux for 1 hour. The essential oil and the water extract are collected. The residue is combined with other five herbs including *Fructus Forsythiae suspensae* (5g), *Fructus Arctii Lappae* (3g), *Semen Sojae Preparatum* (2.5g), *Herba Lophatheri Gracilis* (2g), and *Radix Glycyrrhizae Uralensis* (2.5g) and then extracted twice with 10-fold of milli-Q water under reflux for 2 hours. The supernatant is collected and combined with the previous water extract. The resulting extract is then lyophilized under reduced pressure. The dried paste is combined with *Flos Lonicerae Japonicae* (5g) and *Radix Platycodi Grandiflori* (3g) and then extracted three times with 10-fold of MeOH. Around 5g of MeOH extract is obtained.

*Fractionation of YQS Extract for Anti-viral Bioassay*

The MeOH extract of YQS is fractionated by reversed-phase HPLC (Lichrospher 100 RP C18 EC 5 μm, 250×4.6 mm ID), using a gradient elution from 10% acetonitrile ($CH_3CN$) to 90% $CH_3CN$ at a flow rate of 1 mL/min.

Peak detection is achieved using an Agilent 1200 series of fast scanning Photo-diode Array detector set at 210, 254 and 280 nm. A total of 5 fractions are obtained.

*Fractionation of YQS Extract for IL-1β bioassay*

The YQS extract is fractionated by reversed-phase HPLC (Lichrospher 100 RP C18 EC 5 μm, 250×4.6 mm ID) using a gradient elution from 10% acetonitrile ($CH_3CN$) to 90% $CH_3CN$ at a flow rate of 1 mL/min.

Peak detection is achieved using an Agilent 1200 series of fast scanning Photo-diode Array detector set at 210, 254 and 280 nm. A total of 13 fractions are obtained.

*Preparation of Human Influenza Viruses*

Human influenza H1N1 virus (A/HK/54/98 (oseltamivir-sensitive strain) and A/Vicotria/07159200/07 (oseltamivir-resistant strain), H9N2 (A/Quail/HK/G1/97), and H3N2 (A/H3N2/1174/99) are prepared as described in Lee *et al.* 2005 and Mok *et al*. 2007 $_{(14, 16)}$, which are incorporated by reference in their entirety.

To briefly illustrate, first, the viruses are isolated from human beings. The isolated human influenza H1N1 virus (A/HK/54/98 (oseltamivir-sensitive strain) and A/Vicotria/07159200/07 (oseltamivir-resistant strain), H9N2 (A/Quail/HK/G1/97), and H3N2 (A/H3N2/1174/99) are cloned by limiting dilution. Seed virus stocks are prepared in MDCK (Madin-Darby canine kidney) cells.

*Viral Infection*

MDCK cells and macrophages are infected with the indicated viruses at a multiplicity of infection (m.o.i.) of 0.2 or 2 for 30 min at 37 °C. The supernatant containing the virus inoculum is then removed, and the cells are incubated in MEM or macrophage serum-free medium (Invitrogen).

*Determination of Tissue Culture Infective Dose ($TCID_{50}$)*

Prior to TCID assays, MDCK cells are seeded at $2 \times 10^4$ cells per well on the 96-well plates. Culture supernatants are harvested from viral-infected cells at 48 hour post-infection. Serial 2-fold dilutions of the supernatant samples are prepared, and the diluted samples are incubated with MDCK cells for 1 hour for viral adsorption.

The virus inoculum is then removed. Cells are washed once and replenished with MEM supplemented with 1μg/ml TPCK-treated trypsin. After four days of incubation, cells are fixed with 10% formaldehyde for 30 min and stained with 0.5% crystal violet to determine viral-induced cytopathic effects. $TCID_{50}$ titers are calculated using the Reed-Muench formula.

*Immunocytochemical Staining*

At the indicated time points after infections, cells are fixed and viral nucleoprotein and M1 protein are stained with the Influenza Detection Kit (Oxoid) according to the manufacturer's protocol.

*Protein Extraction and Western Blot Analysis*

Whole-cell lysates are prepared with total lysis buffer (50 mM Tris- HCl, pH 7.4, 150mMNaCl, 50mMNaF, 10mMb-glycerophosphate, 0.1mMEDTA, 10% glycerol, 1% Triton X-100) containing a protease inhibitor cocktail. For Western blot analysis, 10 μg protein is heat denatured in a sample buffer (125mMTris, pH 6.8, 4% sodium dodecyl sulfate, 20% glycerol, 5% beta-mercaptoethanol, 0.01% bromophenol blue), separated by 10% sodium dodecyl sulfate-polyacrylamide gel electrophoresis, and transferred onto a nitrocellulose membrane for assaying protein levels with ECL™ Western Blotting Detection Reagents (GE Healthcare) solution.

*Transmission Electron Microscopy*

At 18 hour post-infection, cells are fixed with 2.5% glutaraldehyde in the cacodylate buffer (0.1 M sodium cacodylate-HCl buffer, pH 7.4) for 15 min on ice. Cells are then scraped from the culture dish, and the pellet is harvested

by centrifugation at 5000 rpm for 3 min. Cells are fixed with 2.5% glutaraldehyde in cacodylate buffer at 4°C overnight, and then resuspended in cacodylate buffer with 0.1 M sucrose. After washings with cacodylate buffer, cells are fixed in 1% osmium tetroxide in cacodylate buffer for 30 min at room temperature. Cells are washed three times and then dehydrated with graduated ethanol. After two washings with propylene oxide, cell pellets are embedded in epoxy resin. Ultrathin sections with a thickness of 100 nm are cut. Ultrastructural features of cells are examined under a transmission electron microscope (Philips EM208S).

*Quantitative Reverse Transcription-PCR Analyses*

Total RNA are extracted with TRIzol reagent (Invitrogen) according to the manufacturer's instructions. The cDNA is synthesized from total RNA with oligo(dT) primers and Superscript II reverse transcriptase (Invitrogen). The levels of mRNA encoding COX-2 or IL-1β are assayed with TaqMan gene expression assays.

*Prostaglandin $E_2$ Assay*

At 48 hours post-infection, cell culture supernatants are harvested and prostaglandin $E_2$ (PGE2) levels in the supernatant are measured by Prostaglandin $E_2$ EIA Kit (Cayman) according to manufacturer's instructions.

EXAMPLES

Following are examples that illustrate procedures for practicing the invention. These examples should not be construed as limiting.

EXAMPLE 1 - EXTRACTION AND IDENTIFICATION OF FORSYTHOSIDE A AND JACARNONE

Light yellowish powders are obtained by repeated purification of the MeOH extracts prepared from *Fructus Forsythiae* using reversed-phase HPLC. The detailed procedures are summarized in Figure 1.

With bioactivity guided purification using sequential HPLC, an antiviral molecule designated as Compound A1 is eluted at approximately 6.8 min as a single compound (>95% purity) with UV absorbance maximized at 220, 290 and 330 (Fig. 2). The $^{13}$C NMR spectra of Compound A1, as shown in Figure 4, display signals at δ131.5 (C-1), 116.5 (C-2), 146.2 (C-3), 144.8 (C-4), 117.2 (C-5), 121.4 (C-6), 72.4 (C-α), 36.8 (C-β), 104.6 (C-1'), 75.3 (C-2'), 76.0 (C-3'), 72.5 (C-4'), 74.9 (C-5'), 67.8 (C-6'), 102.4 (C-1"), 72.1 (C-2"), 72.4 (C-3"), 74.1 (C-3"), 70.0 (C-4"), 18.1 (C-5"), 127.8 (C-1'"), 115.2 (C-2'"), 147.0 (C-3'''), 149.9 (C-4'''), 116.6 (C-5'''), 123.2 (C-6'''), 147.7 (C-7'''), 114.8 (C-8'''), 168.4 (C-9'''). Compound A1 shows an $[M-H]^-$ ion peak at m/z 623 in its HR-ESI-MS.

In comparison of their spectroscopic data with the data reported in the literature, Compound A1 is identified as forsythoside A. The chemical structure of forsythoside A is shown in Figure 6A. The % content of forsythoside A in the initial dried herb, *Fructus forsythiae,* is 1.24% (w/w). 2.8 mg of yellow amorphous powder (Compound D1) is obtained by repeated purification of the MeOH extracts prepared from *Fructus Forsythiae* using reversed-phase HPLC. The detailed procedures are summarized in Figure 1. With bioactivity guided purification using sequential HPLC, a molecule that inhibits COX-2 expression is eluted at approximately 10.75 min as a single compound (>95% purity) with UV absorbance maximized at 230 (Fig. 3). The $^{13}$C NMR spectra of Compound D1 displays the signals at δ45 (C-7), 52 (C-9), 68 (C-4), 128 (C-2, C-6), 152 (C-3, C-5), 171 (C-8), 187 (C-1) (Fig. 5). Compound D1 shows the mass to charge (m/z) ratio at 182, 169, 150, 122, 109 (100), 94, 81, 74, 69, 43. By comparing its spectroscopic data to the reported data from the literature, Compound D1 is identified as jarcaranone, and its chemical structure is shown in Figure 6B (Ogura *et al*., 1976). The % content of jacaranone in dried herb is 0.036% (w/w).

EXAMPLE 2 - DETERMINATION OF CYTOTOXICITY OF FORSYTHOSIDE A

After identification of the inhibitory effects of forsythoside A (Compound A1) on the influenza virus replication, the cytotoxicity of forsythoside A (Compound A1) is examined as follows. Briefly, Madin-Darby canine kidney (MDCK) cells are seeded in a 24-well plate at a density of $1x10^5$ cells/well and incubated for 18 hours prior to the addition of forsythoside A (Compound A1) at a concentration of 100ug/ml, or dimethylsulfoxide (DMSO) in the control.

After incubation at 37°C with 5% $CO_2$ for 48 hours, cell viability is measured by the Thiazolyl Blue Tetrazolium Bromide (MTT) assay. First, MTT is added to cells at a final concentration of 0.1mg/ml. After incubation for 90 minutes, the culture supernatant is removed and each well is treated with isopropanol for cell fixation. The MTT metabolic product, formazan, is dissolved in isopropanol for five minutes with continuous shaking. The optical densities of formazan and background are measured at 560nm and 670nm, respectively.

The cytotoxicity index is calculated as follows:

$$(\lambda 560_{sample1} - \lambda 670_{sample1}) / (\lambda 560_{DMSO} - \lambda 670_{DMSO}).$$

The results, as shown in Table 1, reveal that forsythoside A (Compound A1) does not exhibit significant cytotoxicity on MDCK cells.

Table 1. Cytotoxicity of Forsythoside A

| | Cytotoxicity index |
|---|---|
| Compound A1 (100ug/ml) | 1.02 |
| DMSO | 1.0 |

EXAMPLE 3 - INHIBITORY EFFECTS OF FORSYTHOSIDE A ON VIRUSES

To determine the inhibitory effects of forsythoside A (Compound A1) on viruses, MDCK cells are infected with human influenza viruses, and a viral titer ($TCID_{50}$) bioassay is performed according to the procedures illustrated as follows.

Briefly, MDCK cells are seeded at $1\times10^5$ cells/well on a 24-well plate, and infected with human influenza viruses including H1N1 (A/HK/54/98), an oseltamivir-sensitive strain; H1N1 (A/Vicotria/07159200/07) (H1N1-R), an oseltamivir-resistant strain; H9N2 (A/Quail/HK/G1/97); and H3N2 (A/H3N2/1174/99) at a multiplicity of infection (m.o.i.) of 2 for 30 minutes, respectively. After that, cells are washed with PBS once to remove non-absorbed viruses, and treated with forsythoside A (Compound A1) at a concentration of 100ug/ml supplemented with minimum essential medium (MEM).

After incubation for 48 hours with forsythoside A (Compound A1) or DMSO, the cell culture supernatant is collected and subject to a viral titer ($TCID_{50}$) assay for measuring the inhibitory effects of forsythoside A (Compound A1) on influenza virus replication. Briefly, a two-fold serially diluted supernatant is added to MDCK cells. Cells are incubated for one hour at 37°C with 5% $CO_2$ for viral adsorption. The cells are then washed with MEM or phosphate-buffered saline to remove non-absorbed virus inoculum, and replenished with fresh MEM supplemented with 1ug/ml N-tosyl-L-phenylalanyl chloromethyl ketone (TPCK)-treated trypsin. Cells are further incubated at 37°C with 5% $CO_2$ for four days, then fixed with 10% formaldehyde for 30 minutes and stained with 0.5% crystal violet for examining the virus-induced cytopathic effects. $TCID_{50}$ titers are calculated using the Reed-Muench formula as is described in Methods and Techniques in Virology, 1993 $_{(17)}$, which is hereby incorporated by reference in its entirety.

The results, as shown in Figure 7, reveal that viral replication in the MDCK cells is suppressed by forsythoside A (Compound A1) at 100 μg/ml. Specifically, the replication of influenza viruses H1N1 (A/HK/54/98), H1N1 (A/Vicotria/07159200/07), H9N2 (A/Quail/HK/G1/97) and H3N2 (A/H3N2/1174/99) is suppressed by forsythoside A (Compound A1) for about 15-, 3-, 24- and 12-fold, respectively when compared with DMSO-treated cells (Figure 7A-D) (Representative results from three experiments). After infecting the cells with H1N1 (A/HK/54/98), Tamiflu (100 μM) was also added to the cells as control (Figure 7E). The virus was suppressed by more than 30-folds when compared with the diluent (PBS)-treated cells (Figures 7E).

EXAMPLE 4 - INHIBITORY EFFECTS OF FORSYTHOSIDE A ON INFLUENZA VIRUSES IN PRIMARY HUMAN BLOOD MACROPHAGES

Primary human blood macrophages are infected with human influenza viruses including H1N1 (A/HK/54/98); H9N2 (A/Quail/HK/G1/97); and H3N2 (A/H3N2/1174/99) at an m.o.i. of 2. Cells are then incubated with forsythoside A (Compound A1, 100 μg/ml) or DMSO for 48 hours. After that, cell culture supernatants are collected and viral titers (TCID50) are measured by titration in MDCK cells. (Representative results from three experiments.) The results, as shown in Figure 8, demonstrate that forsythoside A (Compound A1) possesses anti-influenza virus effects in primary human blood macrophages.

EXAMPLE 5 - INHIBITORY EFFECTS OF FORSYTHOSIDE A ON INFLUENZA VIRUS REPLICATION SHOWN BY IMMUNOCYTOCHEMISTY AND WESTERN BLOT ANALYSIS

This Example demonstrates that forsythoside A suppresses influenza virus replication. MDCK cells are infected with human influenza virus H9N2/G1 (A/Quail/HK/G1/97) at an m.o.i. of 0.2. Cells are then incubated with forsythoside A (Compound A1 at 100μg/ml) or DMSO for 6 or 10 hours. After that, cells are fixed and influenza nucleoproteins and M1 proteins are stained with the Influenza Detection Kit (Oxoid). The numbers of cells expressing viral proteins are counted and percentages of cells expressing viral proteins are calculated.

As shown in Figure 9 (A & B), at 6 hour post-infection (h.p.i.), no significant difference is found when comparing

the percentages of cells expressing viral proteins upon DMSO or forsythoside A (Compound A1) treatments. However, from 6 h.p.i to 10 h.p.i., the number of cells expressing viral proteins increases by around 100% when treated with DMSO; while the percentage only increases by about 15% when treated with forsythoside A (Compound A1). This indicates that fosythoside A (Compound A1) decreases the number of viral-infected cells.

MDCK cells are infected with human influenza virus H9N2/G1 (A/Quail/HK/G1/97) at an m.o.i. of 2 or mock infected. After that, cells are then incubated with forsythoside A (Compound A1) (100μg/ml) or DMSO. Proteins are harvested at 2, 4, 9 or 24 h.p.i.

Expressions of the influenza M1 protein are analyzed by Western blot analysis. Actin is used as a loading control. Expression level of viral M1 proteins is examined and band intensities are measured by Quantity One software (Bio-Rad). The relative intensities are determined by normalizing the M1 intensities to that of actin.

As shown in Figure 9C, the M1 protein expression level is lower in the cells treated with forsythoside A (Compound A1), when compared to those treated with DMSO. At 2 h.p.i., the relative intensity of M1 in DMSO-treated cells is higher than that of the forsythoside A-treated cells by more than four fold. Furthermore, over the period of 2 to 24 h.p.i., the relative intensity increases from 0.13 to 1.7 in DMSO-treated cells. On the contrary, in the cells with forsythoside A (Compound A1) treatment, the relative intensity only reaches 0.39 at 24 h.p.i. These results further show treatment with forsythoside A decreases M1 protein expression and reduces viral loads.

EXAMPLE 6 - INHIBITORY EFFECTS OF FORSYTHOSIDE A ON INFLUENZA VIRUS REPLICATION SHOWN BY ELECTRONIC MICROSCOPY

This Example demonstrates that forsythoside A suppresses influenza virus replication. MDCK cells are infected with human influenza virus H9N2/G1 (A/Quail/HK/G1/97) at an m.o.i. of 2. Cells are then incubated with forsythoside A (Compound A1) (100μg/ml) (C-D) or DMSO (A-B) for 18 hours. Cells are then fixed and ultrastructures are examined by transmission electron microscopy.

As shown in Figure 10, in DMSO-treated cells (A-B), most virions have been completely separated from the cells. In contrast, in forsythoside A (Compound A1)-treated cells, a portion of the virions is still attached to the cells (C-D).

The infected cells are further observed under transmission electron microscopy with higher magnifications. A very small portion of virions, attached by thin neck, are still associated with cells incubated with DMSO (B, arrow heads). In contrast, in forsythoside A-treated cells, the budding virions remain connected to the cell by thick stalks (D, arrows). This indicates a slowed or abnormal budding process due to forsythoside A (Compound A1) treatment.

COMPARATIVE EXAMPLE 7 -INHIBITORY EFFECTS OF JACARANONE ON CYCLOOXYGENASE 2 (COX-2) AND INFLUENZA VIRUS INFECTION

Primary human blood macrophages are infected with H9N2/G1 (A/Quail/HK/G1/97) at an m.o.i. of 2. Cells are then incubated with jacaranone (Compound D1) or DMSO for 3 hours. After that, total RNA are harvested and COX-2 mRNA levels are examined by TaqMan Gene Expression assays.

As shown in Figure 11A, jacaranone (Compound D1) suppresses COX-2 mRNA levels upon H9N2/G1 infection and the suppression occurs in a dose-dependent manner. Jacaranone suppresses COX-2 mRNA level by about 20% at 10μg/ml and by 40% at 20μg/ml.

After incubating the cells with jacaranone (Compound D1) for 48 hours, prostaglandin $E_2$ (PGE2) levels in the cell culture supernatant are measured by the Prostaglandin $E_2$ EIA Kit (Cayman). Figure 11B shows that treating non-infected macrophages with jacaranone (Compound D1) alone does not cause any changes in PGE2 levels, when compared to the mock-treated cells. Infection with H9N2/G1 significantly induces PGE2 levels. Consistent with the RNA results, jacaranone (Compound D1) suppresses PGE2 levels in a dose-dependent manner.

EXAMPLE 8 - INHIBITORY EFFECTS OF YQS FORMULA ON INFLUENZA VIRUS REPLICATION

MDCK cells are infected with H1N1 (A/HK/54/98) or H9N2/G1 (A/Quail/HK/G1/97) at an m.o.i. of 2. Cells are then incubated with the Yin Qiao San (YQS) (100μg/ml) or DMSO for 48 hours. After that, cell culture supernatants are collected and viral titers ($TCID_{50}$) are measured by titration in MDCK cells.

Figure 13A shows that YQS inhibits H1N1 and H9N2/G1 virus replications. In addition to YQS, cells are incubated with YQS fractions (F1 - F5) (100μg/ml) (as shown in Figure 12) or DMSO after being infected with H9N2/G1 (A/Quail/HK/G1/97).

After 48-hour incubation, cell culture supernatants are collected and viral titers ($TCID_{50}$) are measured by titration in MDCK cells. As shown in Figure 13C, fractions F2 - F5 show viral inhibitory effects. Among F2 - F5, fractions F2 and F3 have the strongest anti-viral effects.

EXAMPLE 9 - INDUCTION OF INTERLEUKIN-1BETA BY YQS FORMULA

Primary human blood macrophages are infected with H1N1 (A/HK/54/98) at an m.o.i. of 2 or mock infected. H1N1-infected cells are then incubated with YQS (100μg/ml) or DMSO for 3 hours. After that, total RNA are harvested and interleukin(IL)-1β mRNA levels are examined by TaqMan Gene Expression assays. Figure 15A shows that YQS enhances IL-1β mRNA production induced by H1N1.

Primary human blood macrophages cells are incubated with YQS fractions [S1 - S-13, 100μg/ml] (as shown in Figure 14) or DMSO after being infected with H1N1. Figure 15B shows that H1N1 infection increases IL-1β mRNA level by about 3 folds (DMSO), when compared with the mock-infected cells. Treatment with YQS fraction S11 increases IL-1β level by about 20 fold, when compared with the mock-infected cells.

The terms "a" and "an" and "the" and similar referents as used in the context of describing the invention are to be construed to cover both the singular and the plural, unless otherwise indicated herein or clearly contradicted by context.

Recitation of ranges of values herein are merely intended to serve as a shorthand method of referring individually to each separate value falling within the range, unless otherwise indicated herein, and each separate value is incorporated into the specification as if it were individually recited herein. Unless otherwise stated, all exact values provided herein are representative of corresponding approximate values (e.g., all exact exemplary values provided with respect to a particular factor or measurement can be considered to also provide a corresponding approximate measurement, modified by "about," where appropriate).

The use of any and all examples, or exemplary language (e.g., "such as") provided herein, is intended merely to better illuminate the invention and does not pose a limitation on the scope of the invention unless otherwise indicated. No language in the specification should be construed as indicating any element is essential to the practice of the invention unless as much is explicitly stated.

The description herein of any aspect or embodiment of the invention using terms such as "comprising", "having", "including" or "containing" with reference to an element or elements is intended to provide support for a similar aspect or embodiment of the invention that "consists of", "consists essentially of", or "substantially comprises" that particular element or elements, unless otherwise stated or clearly contradicted by context (e.g., a composition described herein as comprising a particular element should be understood as also describing a composition consisting of that element, unless otherwise stated or clearly contradicted by context).

REFERENCES

1. World Health Organization, HIV/AIDS data and statistics, available at http://www.who.int/hiv/data/en/index.html (last visited on Dec. 8, 2009).

2. World Health Organization, Hepatitis B key facts, available at http://www.who.int/mediacentre/factsheets/fs204/en/ (last visited on Dec. 8, 2009).

3. World Health Organization, Pandemic (H1N1) 2009 - update 77, available at http://www.who.int/csr/don/2009_12_04/en/index.html (last visited on Dec. 8, 2009).

4. Neumann G, Noda T, Kawaoka Y. (2009) Emergence and pandemic potential of swine-origin H1N1 influenza virus. Nature. 2009; 459(7249):931-9.

5. Novel Swine-Origin Influenza A (H1N1) Virus Investigation Team, Dawood FS, Jain S, Finelli L, Shaw MW, Lindstrom S, Garten RJ, Gubareva LV, Xu X, Bridges CB, Uyeki TM (2009) Emergence of a novel swine-origin influenza A (H1N1) virus in humans. N Engl J Med. 360(25):2605-15.

6. *An J, Lee DC, Law AH, Yang CL, Poon LL, Lau AS, Jones SJ.(2009) A novel small-molecule inhibitor of the avian influenza H5N1 virus determined through computational screening against the neuraminidase. J Med Chem. 52(9):2667-72.

7. *Yang CL, Chik SC, Li JC, Cheung BK, Lau AS. (2009) Identification of the bioactive constituent and its mechanisms of action in mediating the anti-inflammatory effects of black cohosh and related Cimicifuga species on human primary blood macrophages. J Med Chem. 52(21):6707-15.

8. *Lee DC, Yang CL, Chik SC, Li JC, Rong JH, Chan GC, Lau AS (2009) Bioactivity-guided identification and cell signaling technology to delineate the immunomodulatory effects of Panax ginseng on human promonocytic U937 cells. J Transl Med. 7:34.

9. Jackson WT, Giddings TH, Jr., Taylor MP, Mulinyawe S, Rabinovitch M, Kopito RR, Kirkegaard K (2005) Subversion of cellular autophagosomal machinery by RNA viruses. PLoS Biol 3: e156.

10. M. Chiara Maiur|, Einat Zalckvar, Adi Kimchi, Guido Kroemer (2007) Self-eating and self-killing: crosstalk between autophagy and apoptosis Nat Rev Mol Cell Biol 8(9):741-52.

11. Cheung CY, Poon LL, Lau AS, Luk W, Lau YL, Shortridge KF, Gordon S, Guan Y, Peiris JS (2002) Induction of

proinflammatory cytokines in human macrophages by influenza A (H5N1) viruses: a mechanism for the unusual severity of human disease? Lancet 360: 1831-7.

12. Guan Y, Poon LL, Cheung CY, Ellis TM, Lim W, Lipatov AS, Chan KH, Sturm-Ramirez KM, Cheung CL, Leung YH, Yuen KY, Webster RG, Peiris JS. (2004) H5N1 influenza: a protean pandemic threat. Proc Natl Acad Sci U S A. 101(21):8156-61.

13. Beigel JH, Farrar J, Han AM, Hayden FG, Hyer R, de Jong MD, Lochindarat S, Nguyen TK, Nguyen TH, Tran TH, Nicoll A, Touch S, Yuen KY; Writing Committee of the World Health Organization (WHO) Consultation on Human Influenza A/H5. (2005) Avian influenza A (H5N1) infection in humans. N Engl J Med. 353(13):1374-85.

14. *Lee DC, Cheung CY, Law AH, Mok CK, Peiris M, Lau AS (2005) p38 mitogen-activated protein kinase-dependent hyperinduction of tumor necrosis factor alpha expression in response to avian influenza virus H5N1. J Virol 79: 10147-54.

15. Hui KP, Lee SM, Cheung CY, Ng IH, Poon LL, Guan Y, Ip NY, Lau AS, Peiris JS. (2009) Induction of proinflammatory cytokines in primary human macrophages by influenza A virus (H5N1) is selectively regulated by IFN regulatory factor 3 and p38 MAPK. J Immunol. 182(2):1088-98.

16. *Mok CK, Lee DC, Cheung CY, Peiris M, Lau AS. (2007) Differential onset of apoptosis in influenza A virus H5N1- and H1N1-infected human blood macrophages. J Gen Virol. 88(Pt 4):1275-80.

17. Methods and Techniques in Virology 1993, edited by Payment P and Trudel M, Marcel Dekker Inc. pp. 32-33.

## Claims

1. An isolated compound being forsythoside A (compound A1)

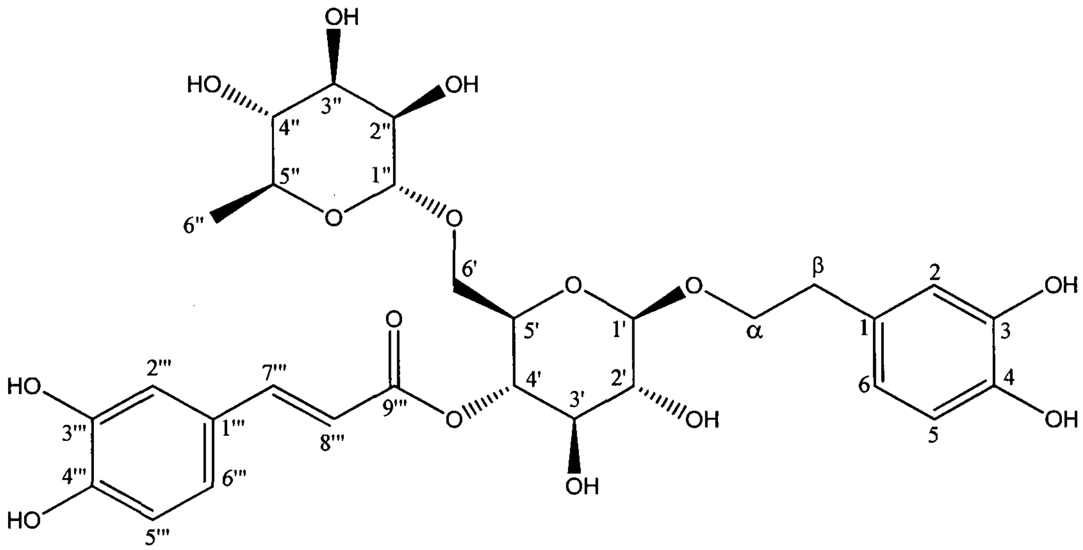

;

or a salt thereof;
for use in a method for preventing and/or treating viral infection caused by influenza virus in a subject, wherein said method comprises administering, to a subject in need of such prevention and/or treatment, an effective amount of said compound, wherein said method is used to prevent and/or treat an infection caused by an oseltamivir-resistant H1N1 influenza virus.

2. The compound for use according to claim 1, wherein said oseltamivir-resistant H1N1 influenza virus is A/Vicotria/07159200/07.

3. The compound for use according to claim 1, wherein the subject is a human.

4. The compound for use according to claim 1, wherein the compound is forsythoside A.

5. The compound for use according to claim 1, wherein said method further comprises administering to the subject a second anti-viral agent selected from the group consisting of zanamivir, peramivir, seltamivir, and any combination thereof.

**6.** The compound for use according to claim 1, wherein said method is used to enhance IL-1β level, or is used to reduce COX-2 level, or is used to reduce PGE2 level.

**7.** The compound for use according to claim 1, wherein said method further comprises a step of determining the level of viral infection in the subject; wherein the determination is optionally made at multiple times to monitor the change over time.

**8.** The compound for use according to claim 1, wherein, in said method, the administration is orally.

**9.** The compound for use according to claim 1, wherein, in said method, the compound is administered in the absence of any other forsythoside compound.

**10.** A therapeutic composition comprising an antiviral amount of an isolated compound , said compound being forsythoside A (compound A1)

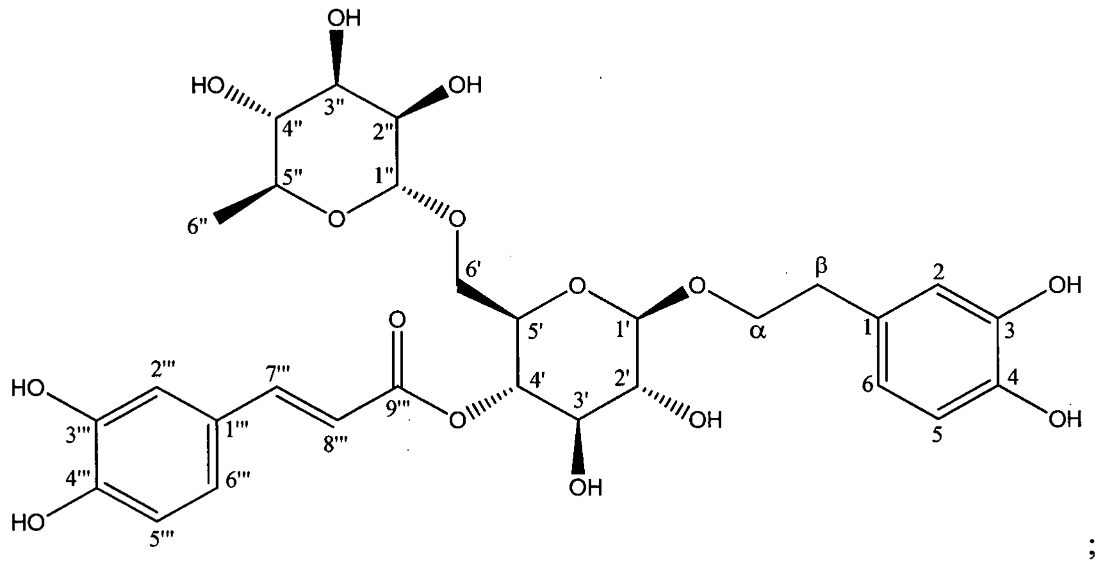

;

or a salt thereof;
for use in a method for preventing and/or treating viral infection caused by influenza virus in a subject, wherein said method comprises administering, to a subject in need of such prevention and/or treatment an effective amount of said compound, wherein said method is used to prevent and/or treat an infection caused by an oseltamivir-resistant H1N1 influenza virus.

**11.** The therapeutic composition for use according to claim 10, wherein said oseltamivir-resistant H1N1 influenza virus is A/Vicotria/07159200/07.

**12.** The therapeutic composition for use according to claim 10, wherein the compound is forsythoside A.

**13.** The therapeutic composition for use according to claim 10, wherein the composition is formulated for oral administration.

**14.** The therapeutic composition for use according to claim 10, which does not contain any other forsythoside compound.

### Patentansprüche

**1.** Isolierte Verbindung, die Forsythosid A (Verbindung A1) ist

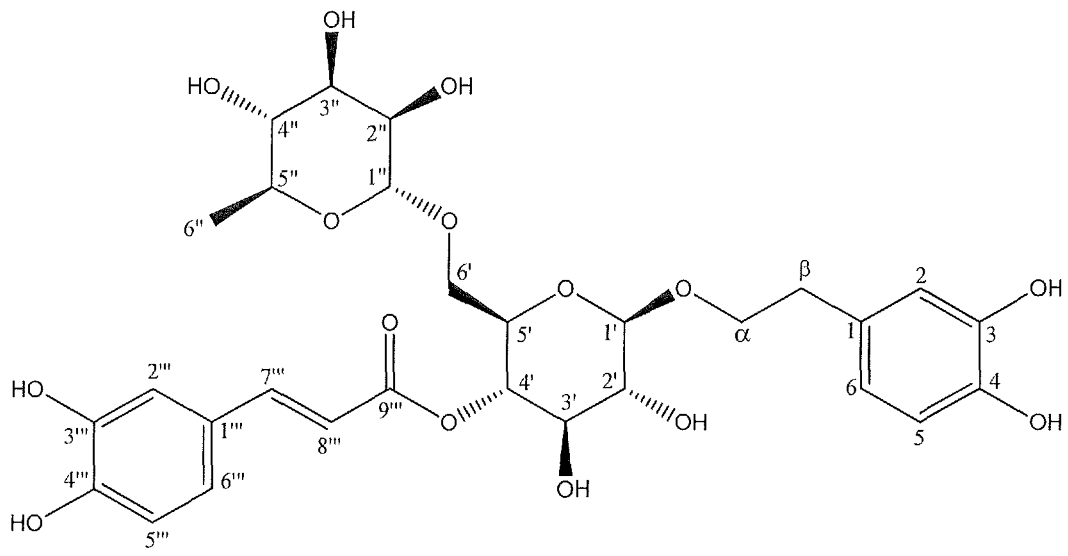

;

oder ein Salz davon;
zur Verwendung in einem Verfahren zum Verhindern und/oder Behandeln viraler Infektion, die durch Influenzavirus verursacht ist, in einem Patienten, wobei das Verfahren umfasst: Verabreichen einer wirksamen Menge der Verbindung an einem Patienten, der solche Verhinderung und/oder Behandlung benötigt, wobei das Verfahren verwendet wird, um eine Infektion zu verhindern und/oder zu behandeln, die durch ein Oseltamivir-resistentes H1N1-Influenzavirus verursacht ist.

2. Verbindung zur Verwendung nach Anspruch 1, wobei das Oseltamivir-resistente H1N1-Influenzavirus A/Vicotria/07159200/07 ist.

3. Verbindung zur Verwendung nach Anspruch 1, wobei der Patient ein Mensch ist.

4. Verbindung zur Verwendung nach Anspruch 1, wobei die Verbindung Forsythosid A ist.

5. Verbindung zur Verwendung nach Anspruch 1, wobei das Verfahren zusätzlich umfasst; Verabreichen eines zweiten antiviralen Mittels, ausgewählt aus der Gruppe, bestehend aus Zanamivir, Peramivir, Seltamivir, und jeder Kombination davon, an den Patienten.

6. Verbindung zur Verwendung nach Anspruch 1, wobei das Verfahren verwendet wird, um das IL-1β Level zu steigern, oder
verwendet wird, um das COX-2 Level zu senken, oder
verwendet wird, um das PGE2 Level zu senken.

7. Verbindung zur Verwendung nach Anspruch 1, wobei das Verfahren zusätzlich einen Schritt umfasst: Bestimmen des Level der viralen Infektion in dem Patienten; wobei die Bestimmung wahlweise mehrere Male durchgeführt wird, um die Änderung über die Zeit zu überwachen.

8. Verbindung zur Verwendung nach Anspruch 1, wobei in dem Verfahren die Verabreichung oral ist.

9. Verbindung zur Verwendung nach Anspruch 1, wobei in dem Verfahren die Verbindung in Abwesenheit einer anderen Forsythosid-Verbindung verabreicht wird.

10. Therapeutische Zusammensetzung, die eine antivirale Menge einer isolierten Verbindung umfasst, wobei die Verbindung Forsythosid A (Verbindung A1) ist

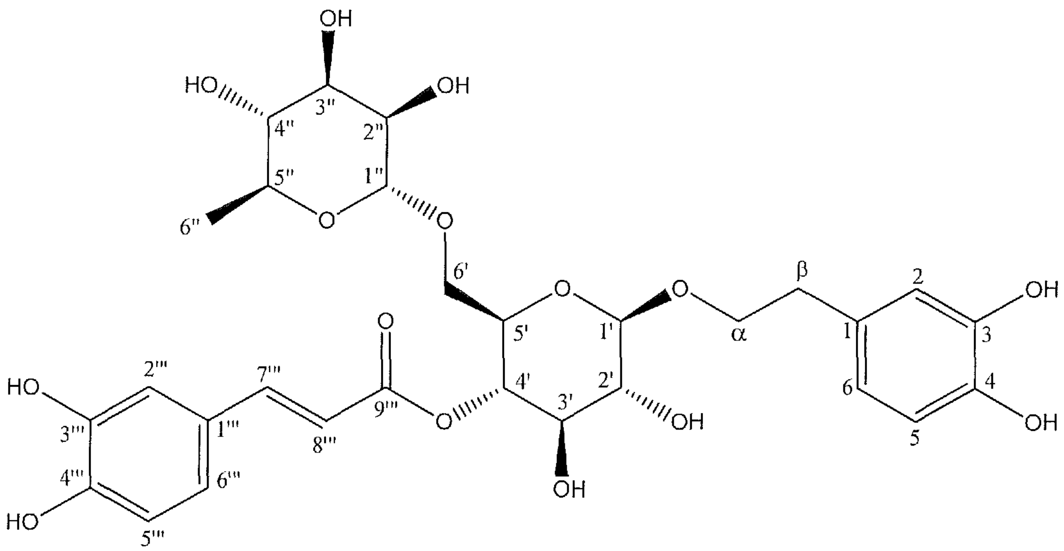

;

oder ein Salz davon;
zur Verwendung in einem Verfahren zum Verhindern und/oder Behandeln viraler Infektion, die durch Influenzavirus verursacht ist, in einem Patienten, wobei das Verfahren umfasst: Verabreichen einer wirksamen Menge einer Verbindung an einen Patienten, der solche Verhinderung und/oder Behandlung benötigt, wobei das Verfahren verwendet wird, um eine Infektion zu verhindern und/oder zu behandeln, die durch ein Oseltamivir-resistentes H1N1-Influenzavirus verursacht ist.

**11.** Therapeutische Zusammensetzung zur Verwendung nach Anspruch 10, wobei das Oseltamivir-resistente H1N1-Influenzavirus A/Vicotria/07159200/07 ist.

**12.** Therapeutische Zusammensetzung zur Verwendung nach Anspruch 10, wobei die Verbindung Forsythosid A ist.

**13.** Therapeutische Zusammensetzung zur Verwendung nach Anspruch 10, wobei die Zusammensetzung für orale Verabreichung formuliert ist.

**14.** Therapeutische Zusammensetzung zur Verwendung nach Anspruch 10, wobei sie keine andere Forsythosid-Verbindung enthält.

## Revendications

**1.** Composé isolé qui est le forsythoside A (composé A1)

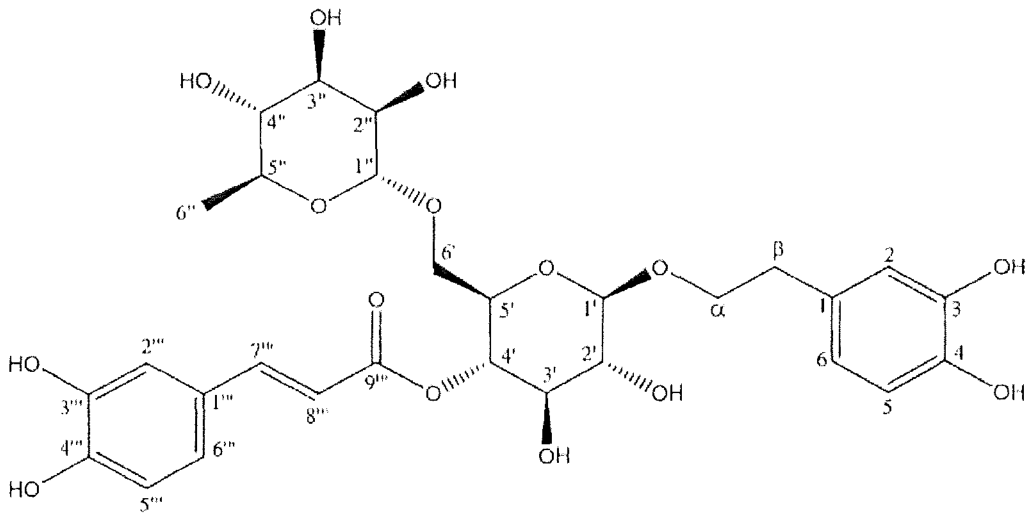

;

ou l'un de ses sels ;
pour une utilisation dans un procédé de prévention et/ou de traitement d'une infection virale provoquée par le virus de la grippe chez un sujet, dans lequel ledit procédé comprend l'administration, à un sujet ayant besoin d'une telle prévention et/ou d'un tel traitement, d'une quantité efficace dudit composé, dans lequel ledit procédé est utilisé pour prévenir et/ou traiter une infection provoquée par un virus de la grippe H1N1 résistant à l'oseltamivir.

2. Composé pour une utilisation selon la revendication 1, dans lequel ledit virus de la grippe H1N1 résistant à l'oseltamivir est A/Victoria/07159200/07.

3. Composé pour une utilisation selon la revendication 1, dans lequel le sujet est un être humain.

4. Composé pour une utilisation selon la revendication 1, dans lequel le composé est le forsythoside A.

5. Composé pour une utilisation selon la revendication 1, dans lequel ledit procédé comprend en outre l'administration au sujet d'un second agent antiviral choisi dans le groupe constitué de zanamivir, péramivir, seltamivir, et l'une quelconque de leurs combinaisons.

6. Composé pour une utilisation selon la revendication 1, dans lequel ledit procédé est utilisé pour amplifier le taux d'IL-1β, ou est
utilisé pour réduire le taux de COX-2, ou est
utilisé pour réduire le taux de PGE2.

7. Composé pour une utilisation selon la revendication 1, dans lequel ledit procédé comprend en outre une étape de détermination du taux d'infection virale chez le sujet ; dans lequel la détermination est effectuée éventuellement à des temps multiples pour surveiller la variation dans le temps.

8. Composé pour une utilisation selon la revendication 1, dans lequel, dans ledit procédé, l'administration est par voie orale.

9. Composé pour une utilisation selon la revendication 1, dans lequel, dans ledit procédé, le composé est administré en l'absence de tout autre composé de forsythoside.

10. Composition thérapeutique comprenant une quantité antivirale d'un composé isolé, ledit composé étant le forsythoside A (composé A1)

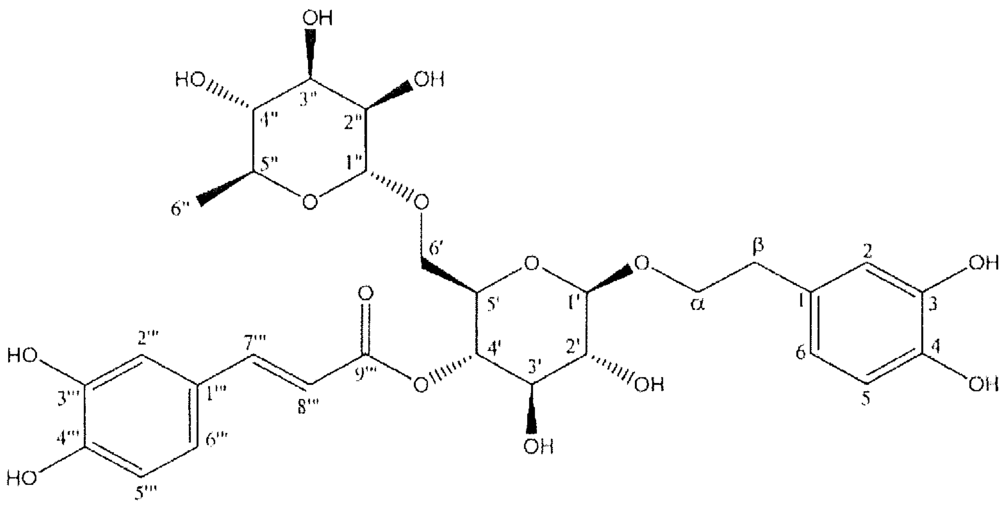

;

ou l'un de ses sels ;

pour une utilisation dans un procédé de prévention et/ou de traitement d'une infection virale provoquée par le virus de la grippe chez un sujet, dans laquelle ledit procédé comprend l'administration, à un sujet ayant besoin d'une telle prévention et/ou d'un tel traitement, d'une quantité efficace dudit composé, dans laquelle ledit procédé est utilisé pour prévenir et/ou traiter une infection provoquée par un virus de la grippe H1N1 résistant à l'oseltamivir.

**11.** Composition thérapeutique pour une utilisation selon la revendication 10, dans laquelle ledit virus de la grippe H1N1 résistant à l'oseltamivir est A/Victoria/07159200/07.

**12.** Composition thérapeutique pour une utilisation selon la revendication 10, dans laquelle le composé est le forsythoside A.

**13.** Composition thérapeutique pour une utilisation selon la revendication 10, dans laquelle la composition est formulée pour une administration orale.

**14.** Composition thérapeutique pour une utilisation selon la revendication 10, qui ne contient aucun autre composé de forsythoside.

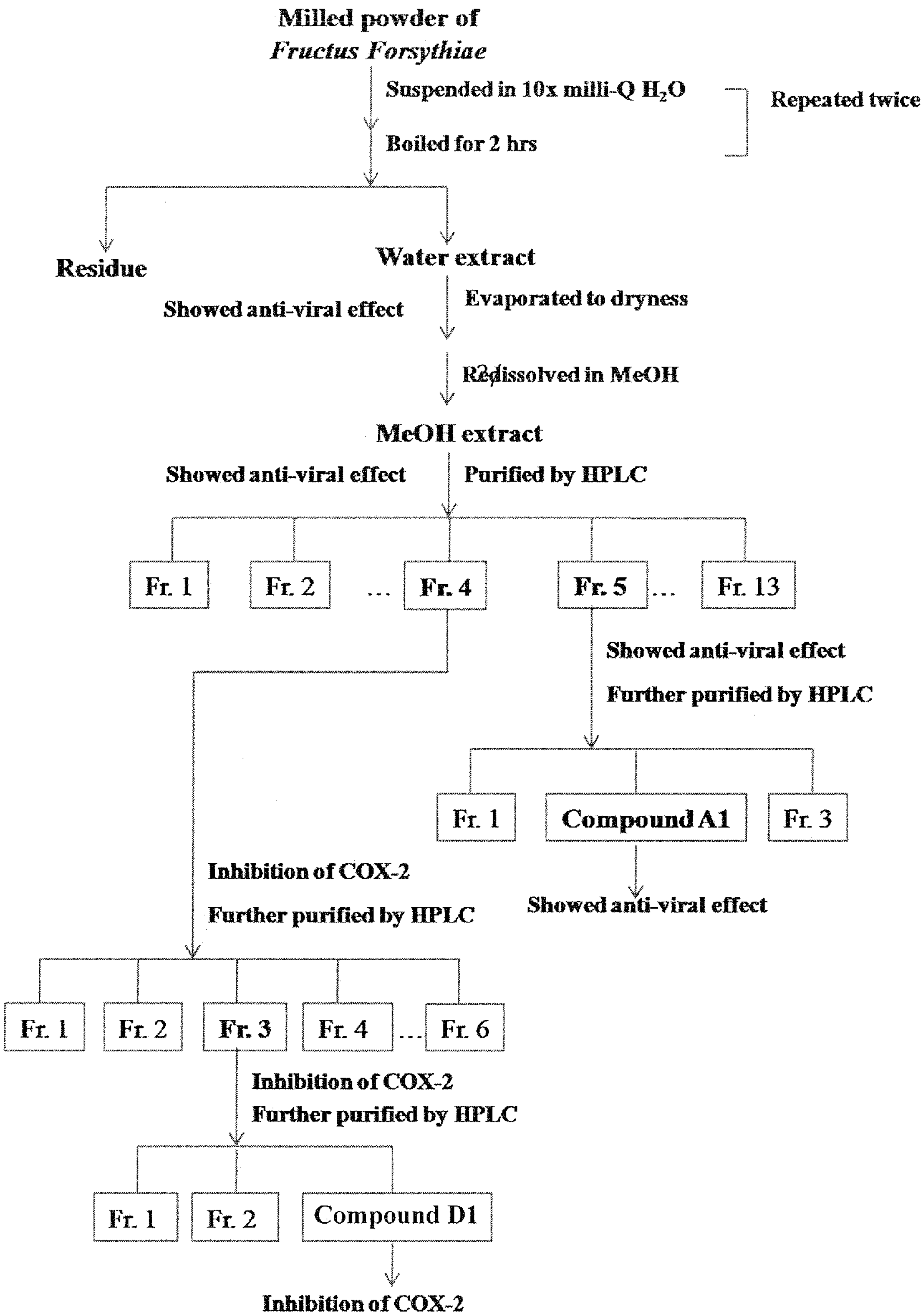
Milled powder of
*Fructus Forsythiae*
Suspended in 10x milli-Q $H_2O$
Boiled for 2 hrs
Repeated twice
Residue
Water extract
Showed anti-viral effect
Evaporated to dryness
Redissolved in MeOH
MeOH extract
Showed anti-viral effect
Purified by HPLC
Fr. 1
Fr. 2
...
Fr. 4
Fr. 5
...
Fr. 13
Showed anti-viral effect
Further purified by HPLC
Fr. 1
Compound A1
Fr. 3
Showed anti-viral effect
Inhibition of COX-2
Further purified by HPLC
Fr. 1
Fr. 2
Fr. 3
Fr. 4
...
Fr. 6
Inhibition of COX-2
Further purified by HPLC
Fr. 1
Fr. 2
Compound D1
Inhibition of COX-2

FIG. 1

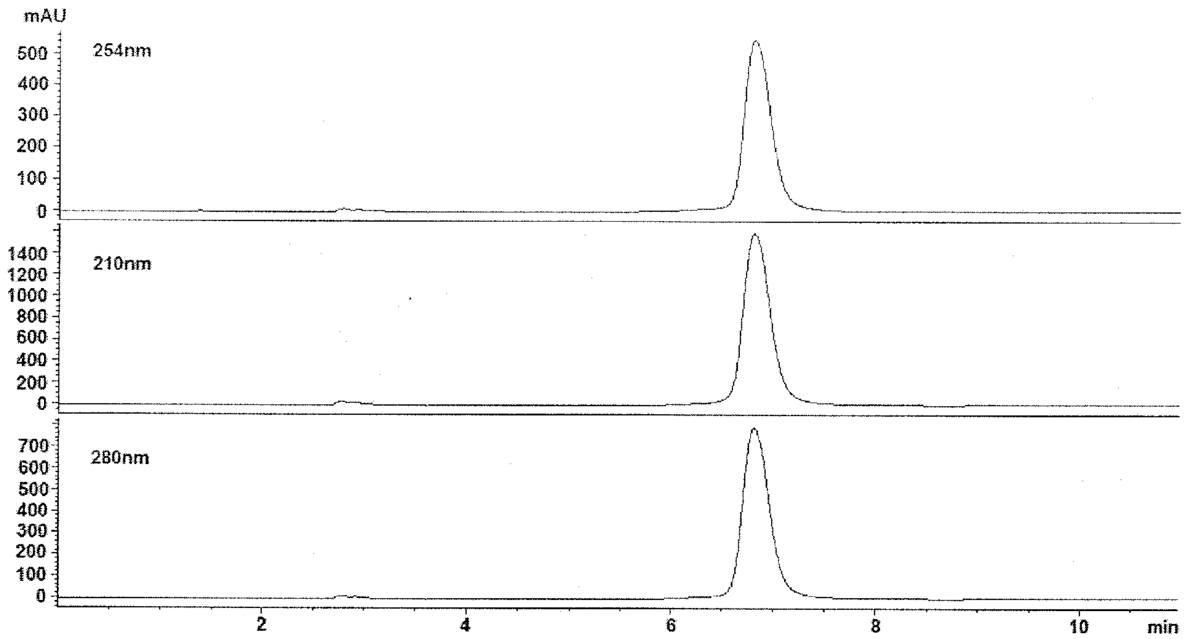
mAU
254nm
500
400
300
200
100
0
210nm
1400
1200
1000
800
600
400
200
0
280nm
700
600
500
400
300
200
100
0
2
4
6
8
10
min

FIG. 2A

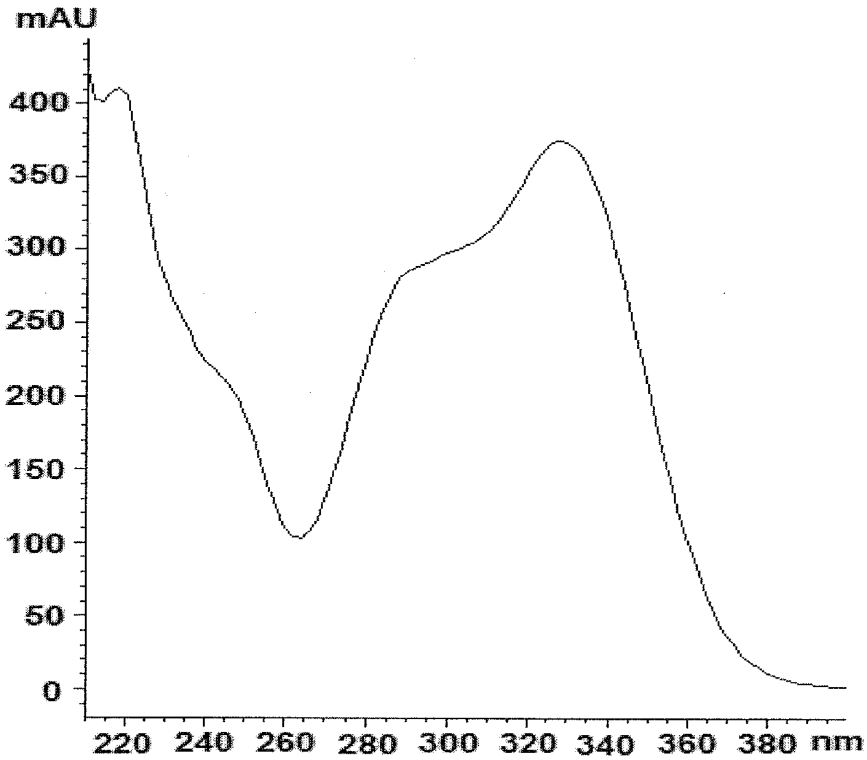
mAU
400
350
300
250
200
150
100
50
0
220
240
260
280
300
320
340
360
380
nm

FIG. 2B

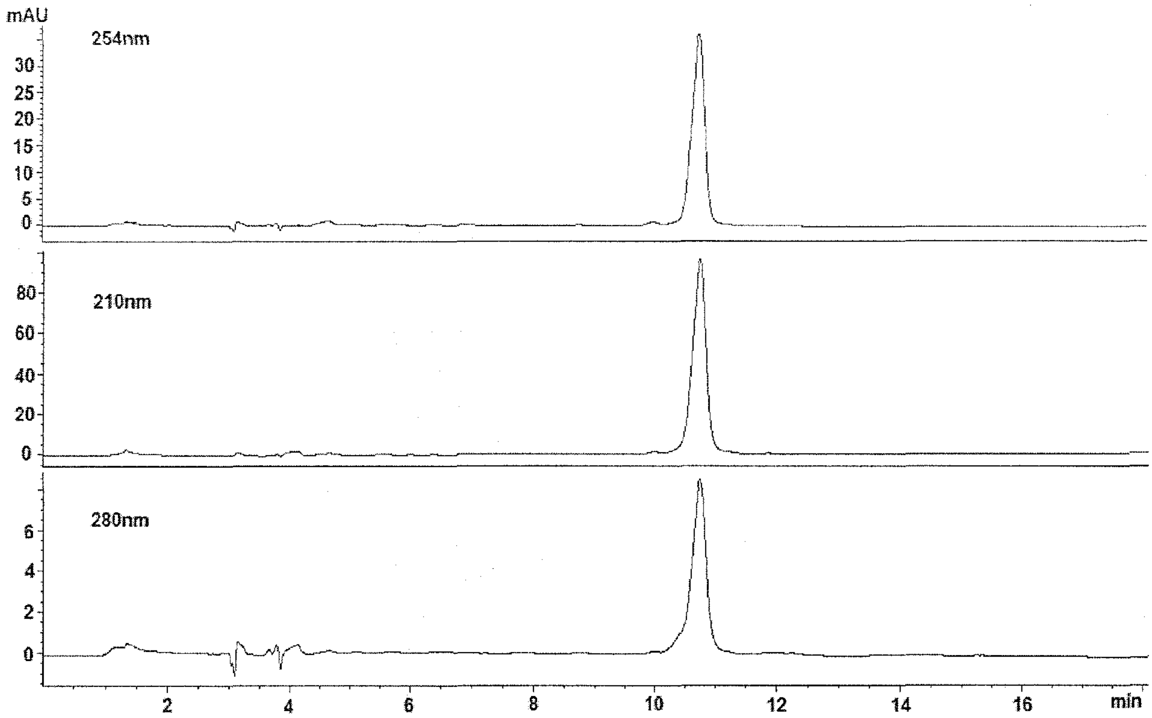

mAU
254nm
30
25
20
15
10
5
0
80
210nm
60
40
20
0
280nm
6
4
2
0
2
4
6
8
10
12
14
16
min


FIG. 3A

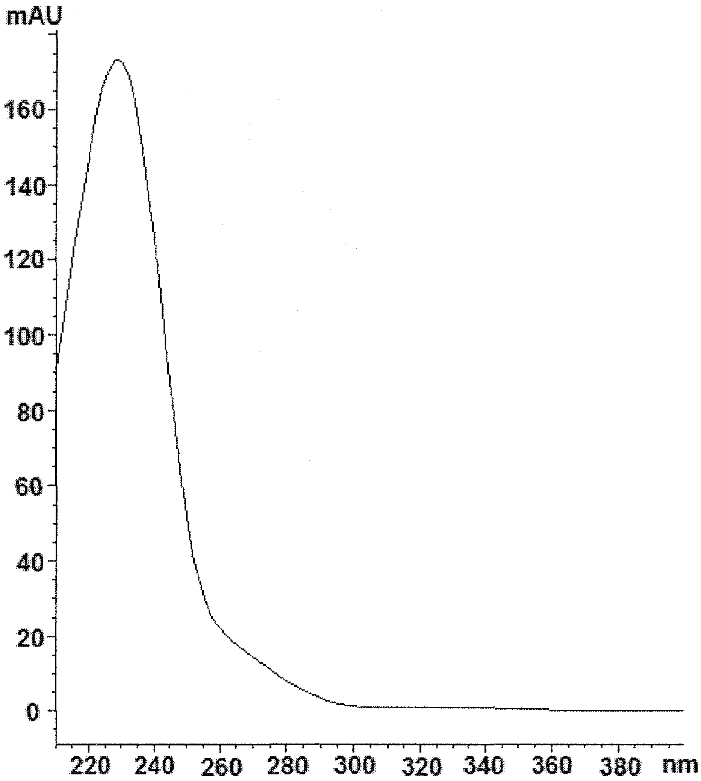

mAU
160
140
120
100
80
60
40
20
0
220
240
260
280
300
320
340
360
380
nm


FIG. 3B

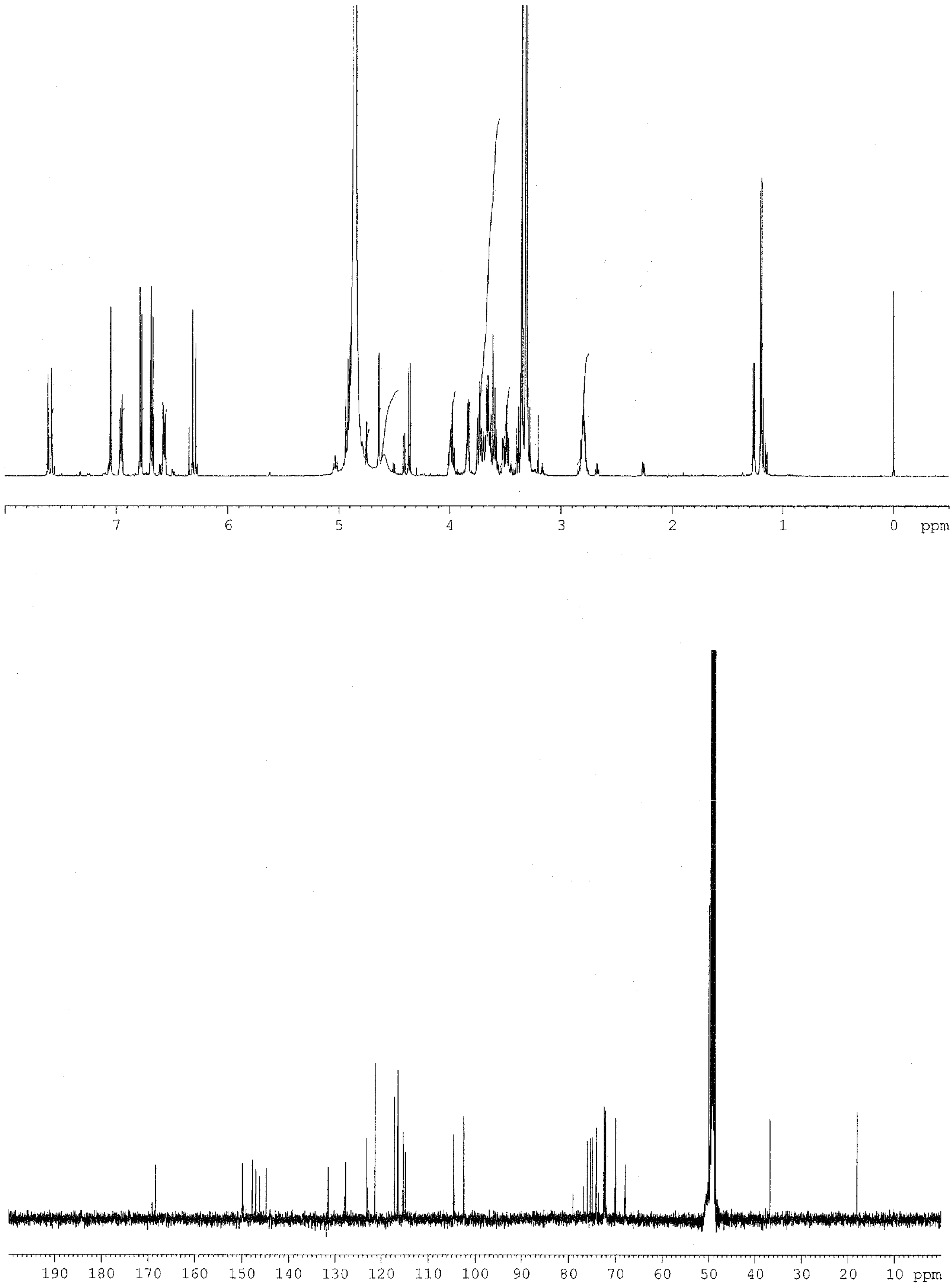
7
6
5
4
3
2
1
0
ppm
190
180
170
160
150
140
130
120
110
100
90
80
70
60
50
40
30
20
10
ppm

FIG. 4

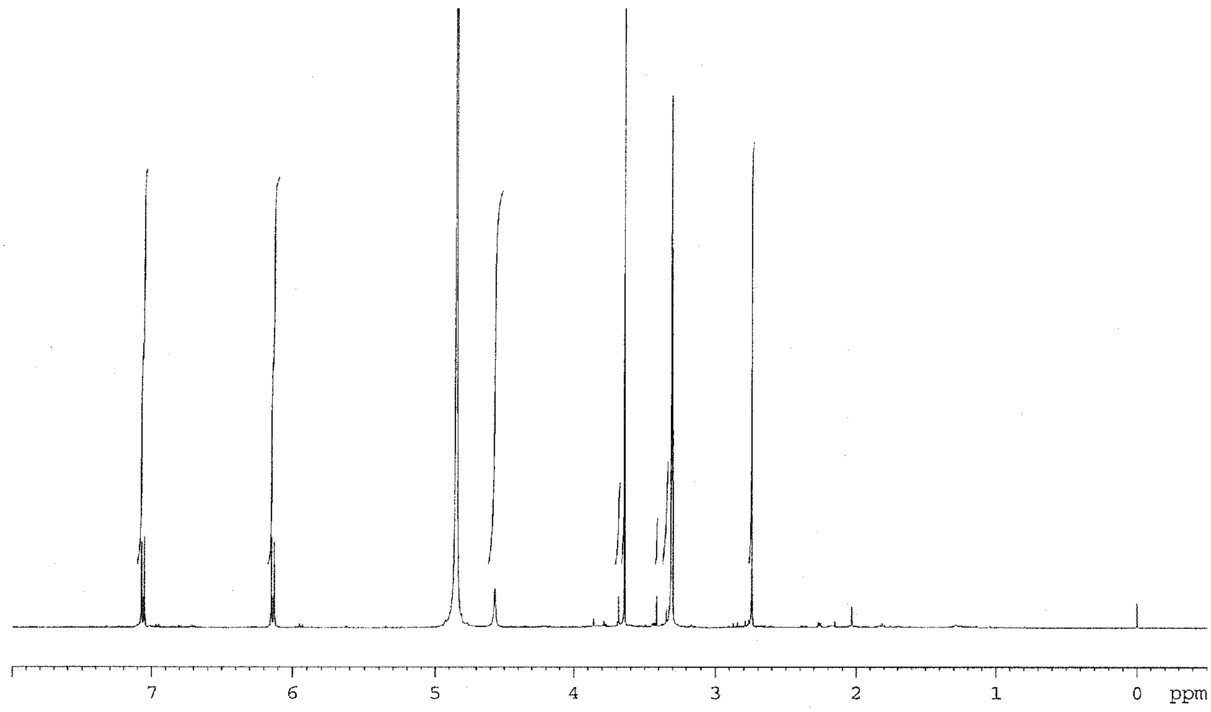


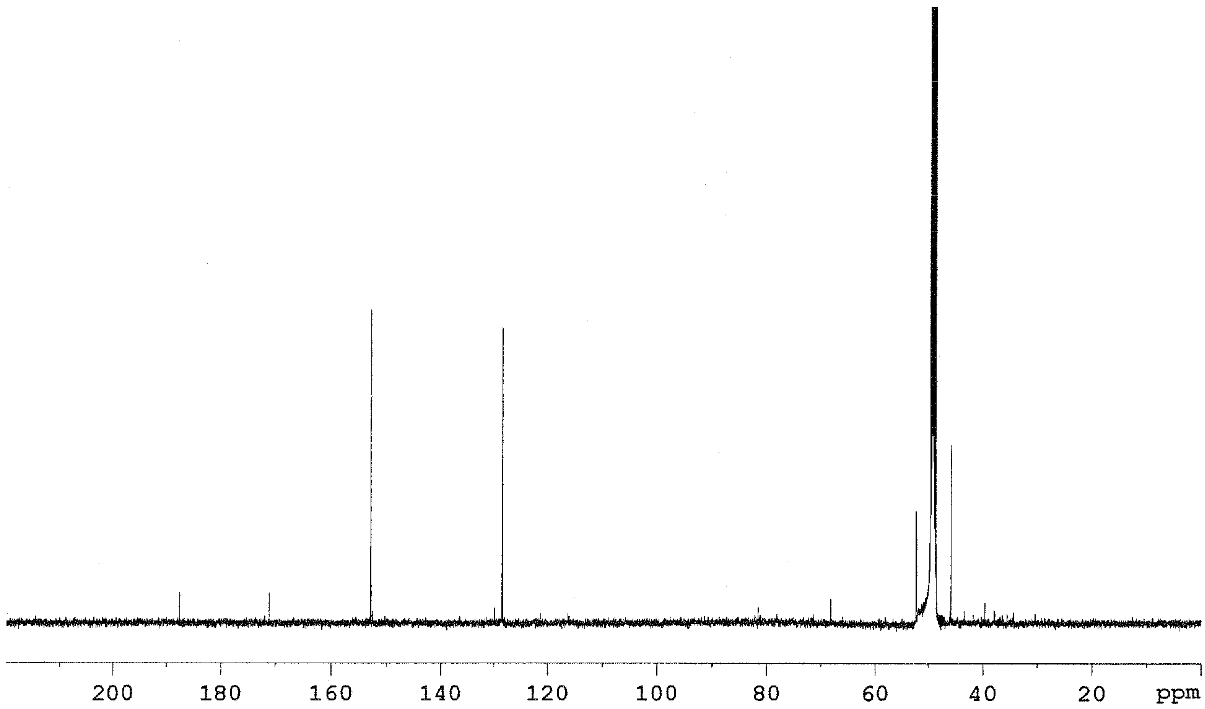


FIG. 5

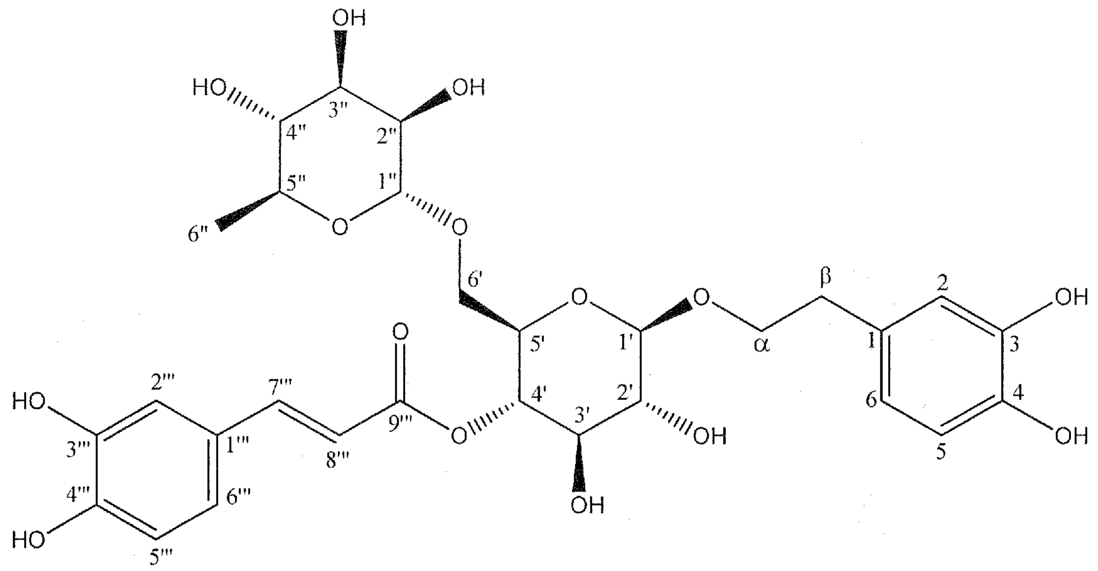

(Compound A1)

FIG. 6A

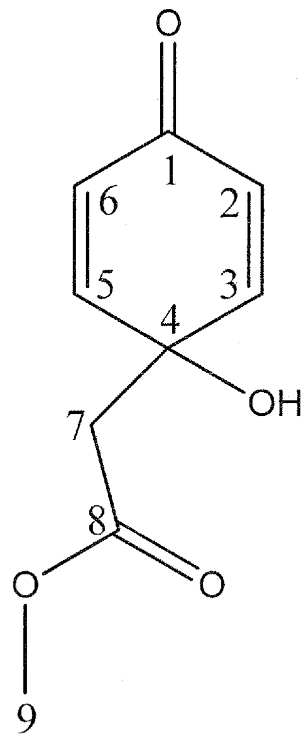

(Compound D1)

FIG. 6B

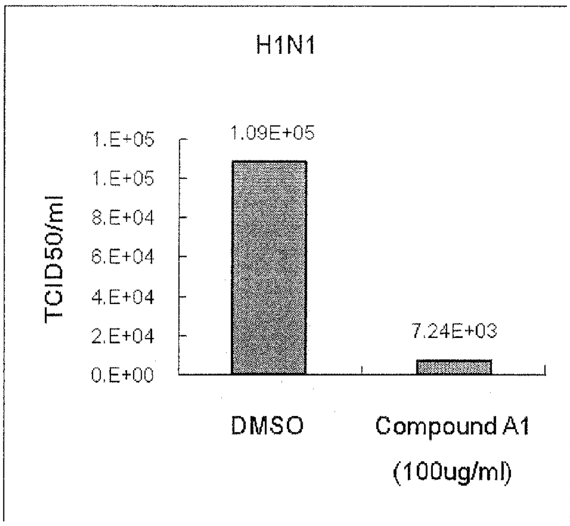


FIG. 7A

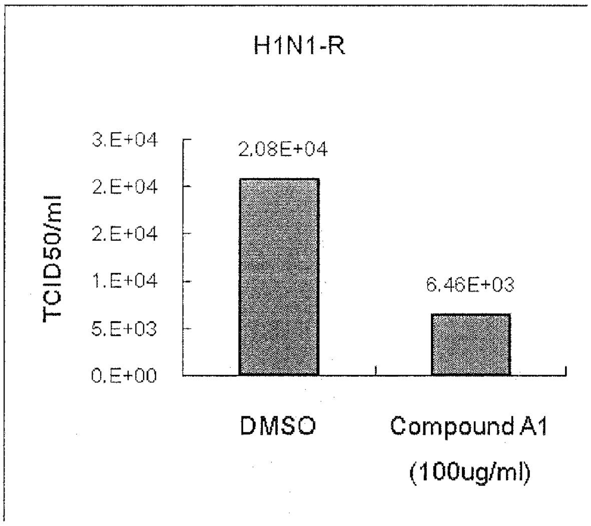


FIG. 7B

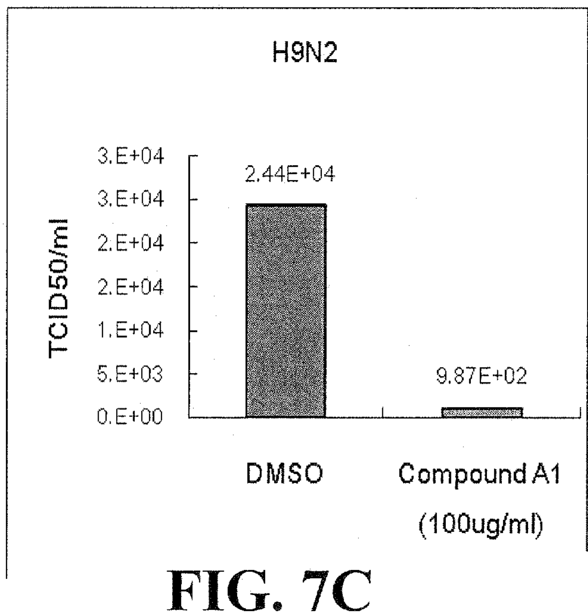


FIG. 7C

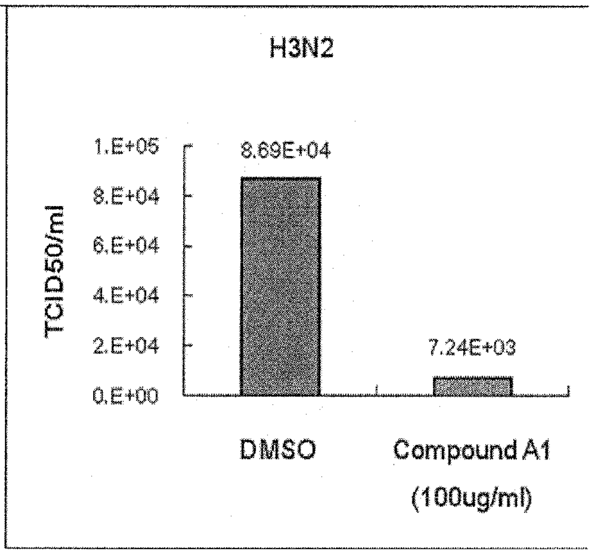


FIG. 7D

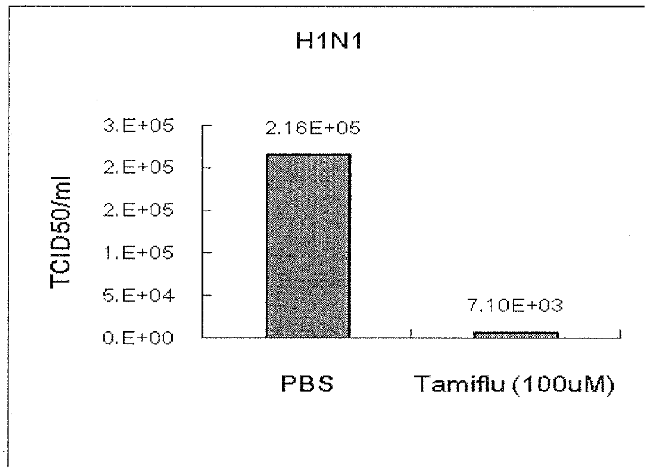


FIG. 7E

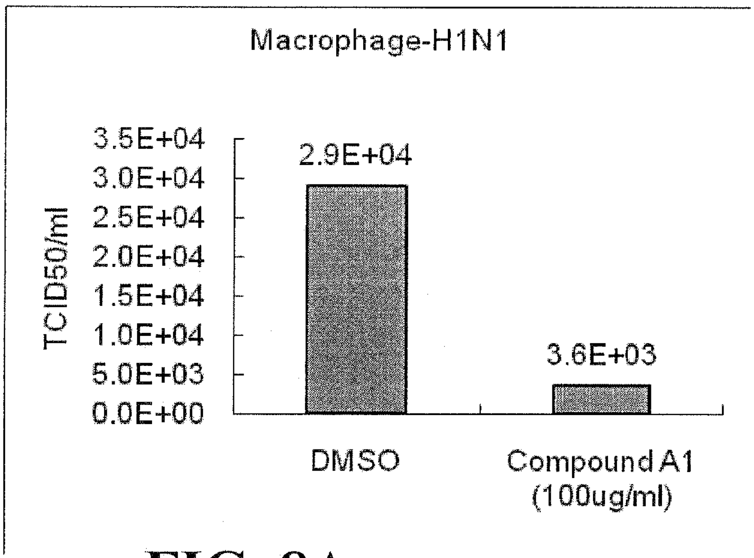


**FIG. 8A**

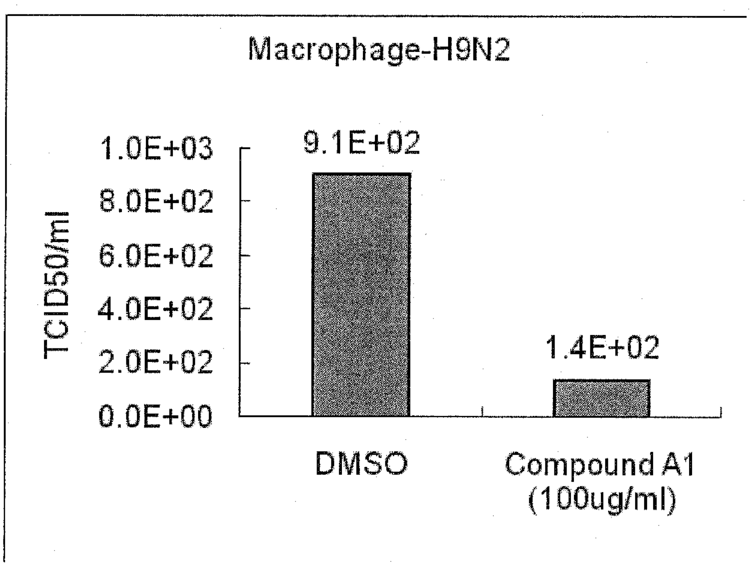


**FIG. 8B**

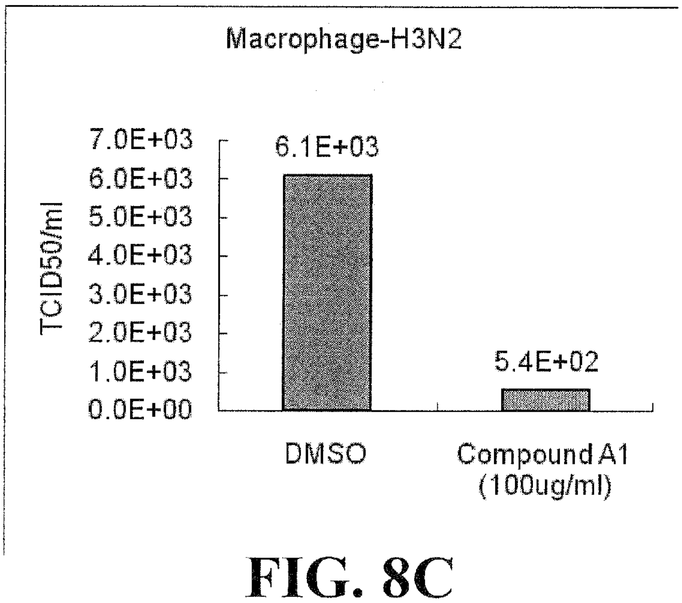


**FIG. 8C**

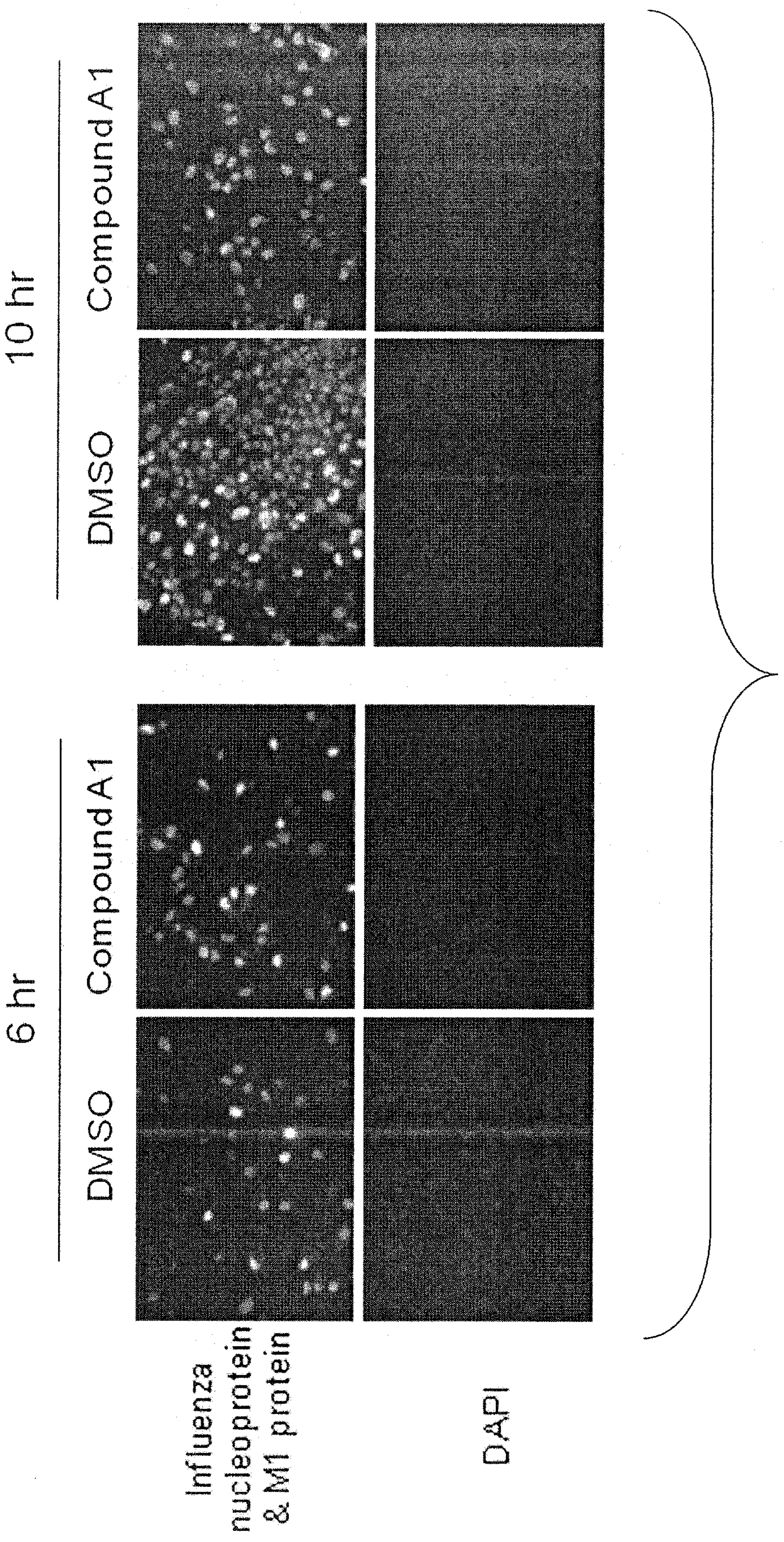
10 hr
6 hr
DMSO
Compound A1
DMSO
Compound A1
Influenza nucleoprotein & M1 protein
DAPI

FIG. 9A

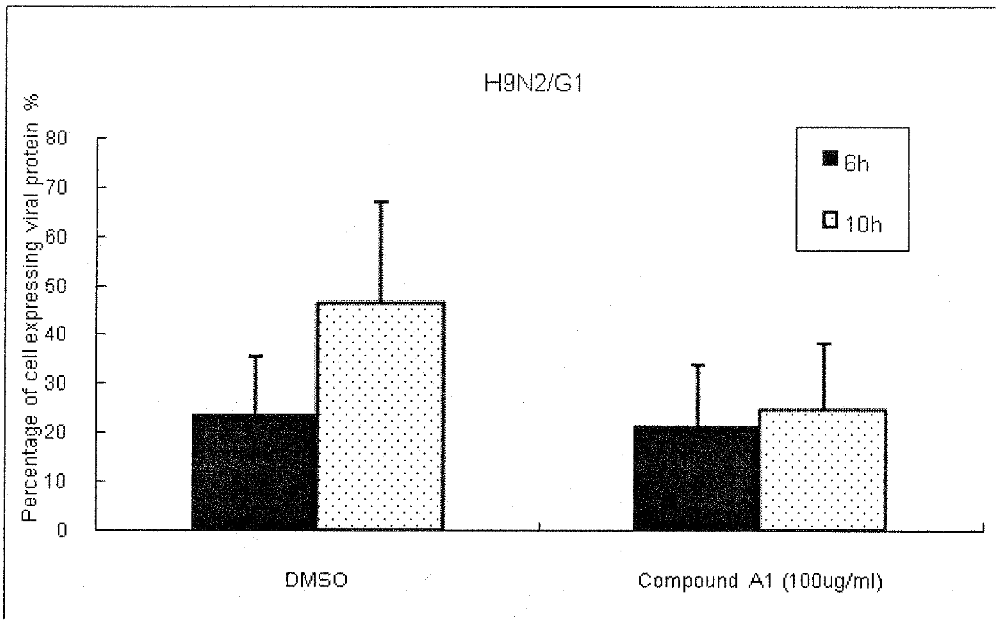
H9N2/G1
Percentage of cell expressing viral protein %
80
70
60
50
40
30
20
10
0
6h
10h
DMSO
Compound A1 (100ug/ml)

FIG. 9B

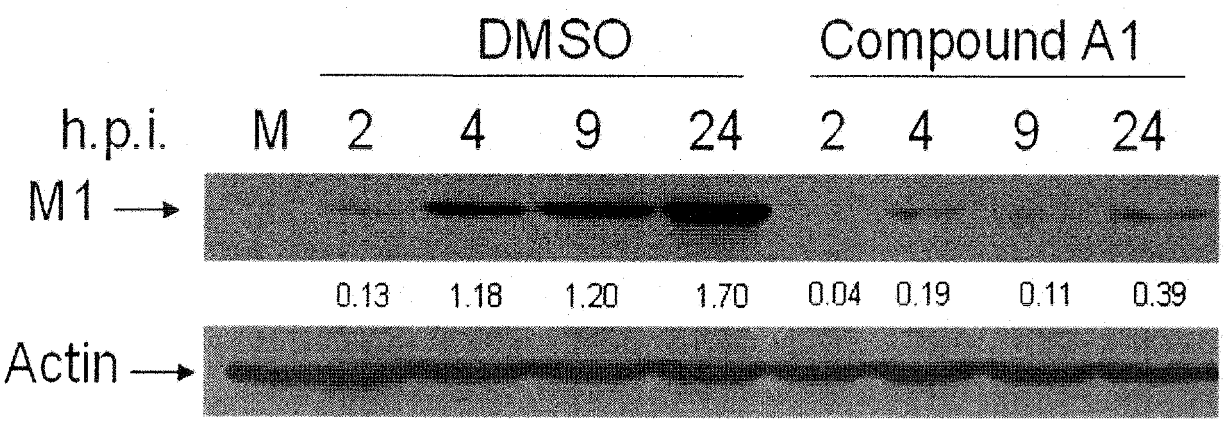
DMSO
Compound A1
h.p.i.
M
2
4
9
24
2
4
9
24
M1
0.13
1.18
1.20
1.70
0.04
0.19
0.11
0.39
Actin

FIG. 9C

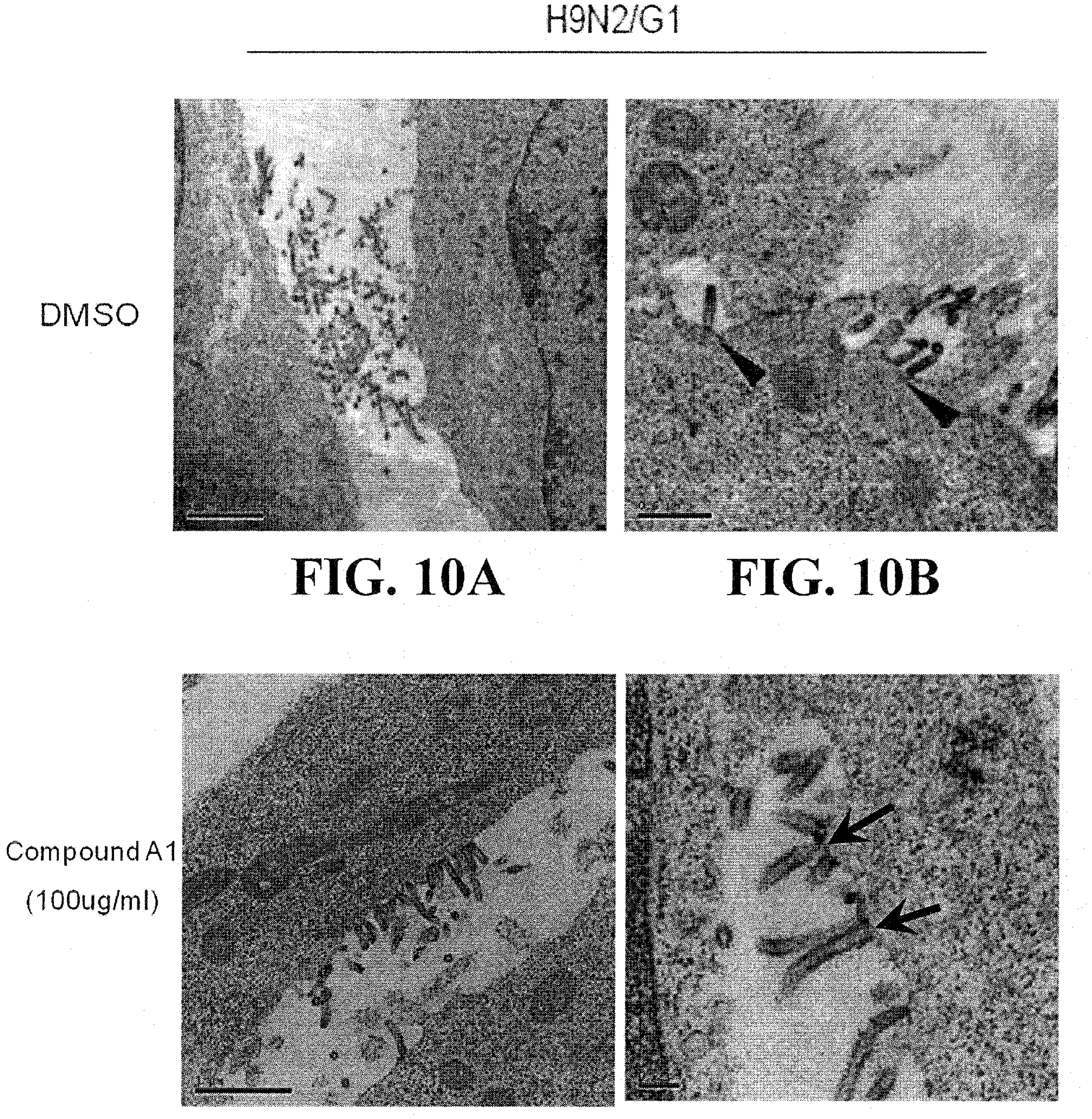


FIG. 10A

FIG. 10B

FIG. 10C

FIG. 10D

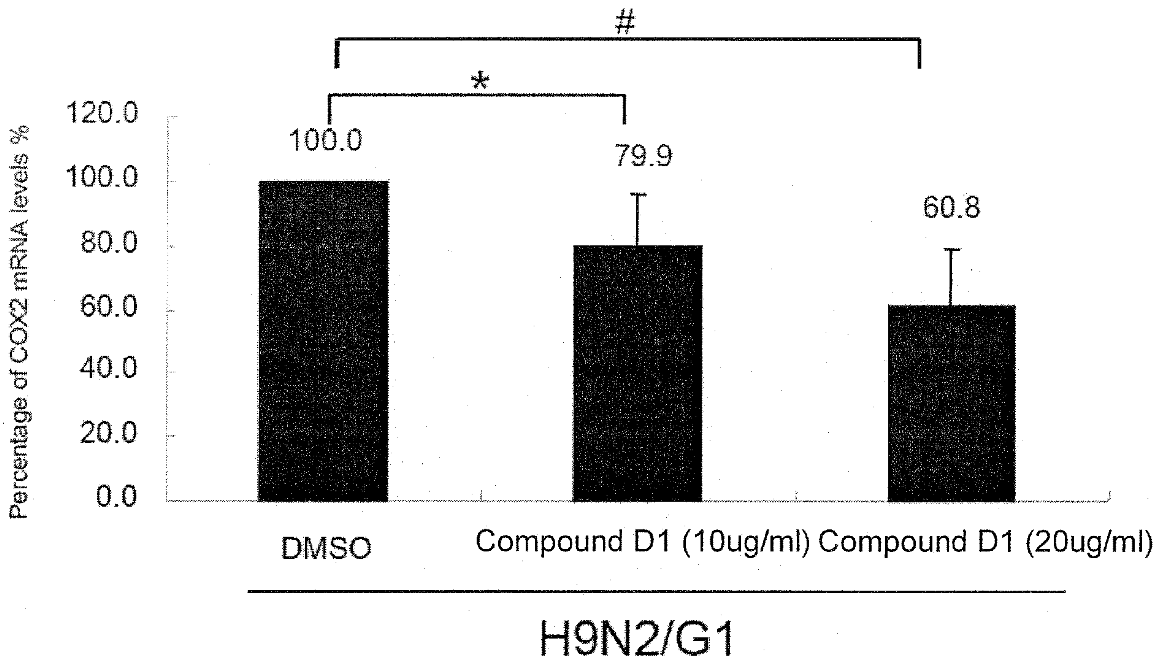


FIG. 11A

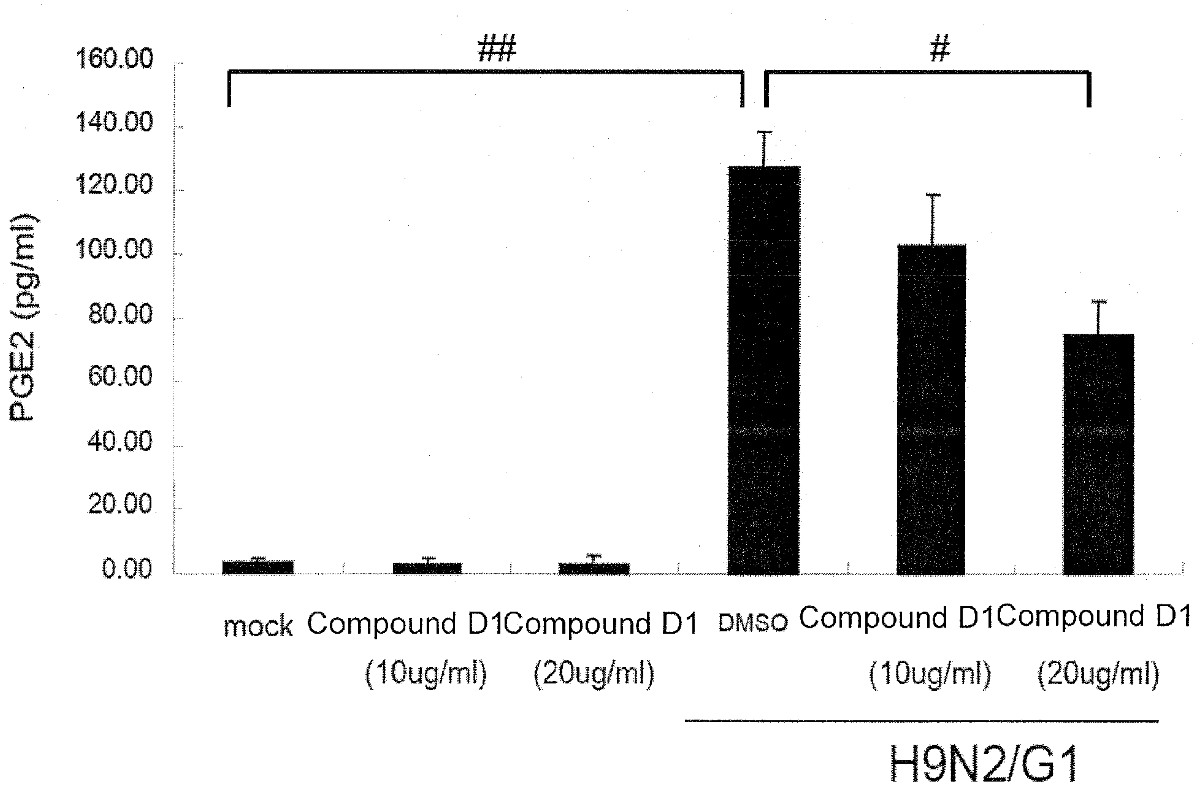


FIG. 11B

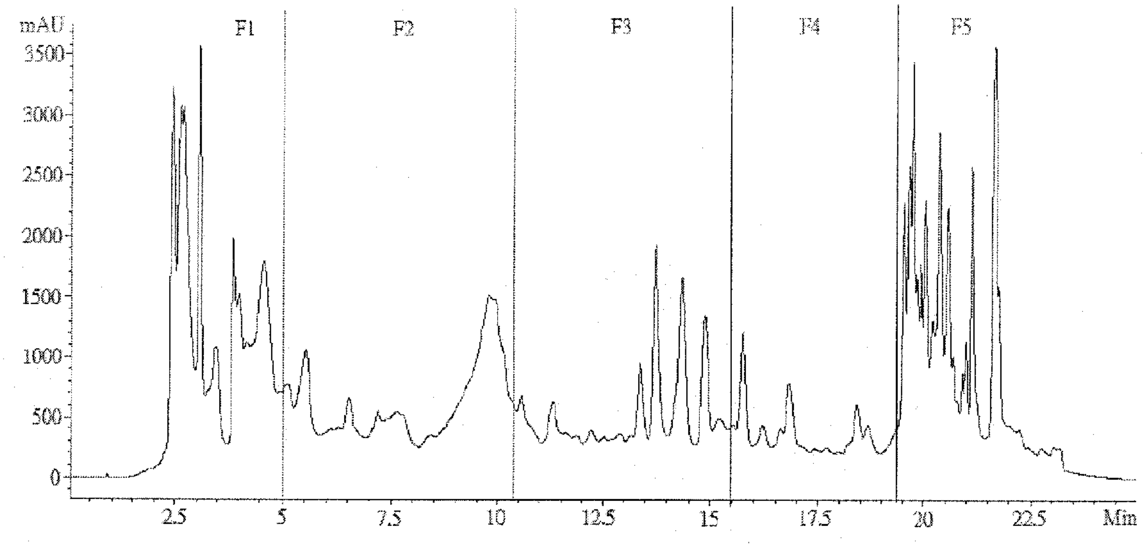


FIG. 12

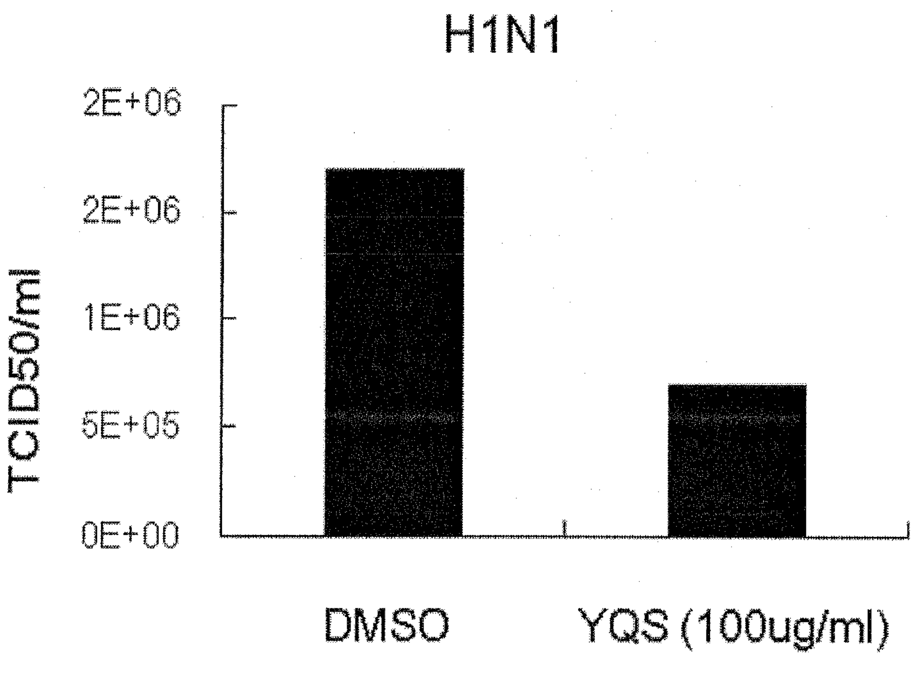


FIG. 13A

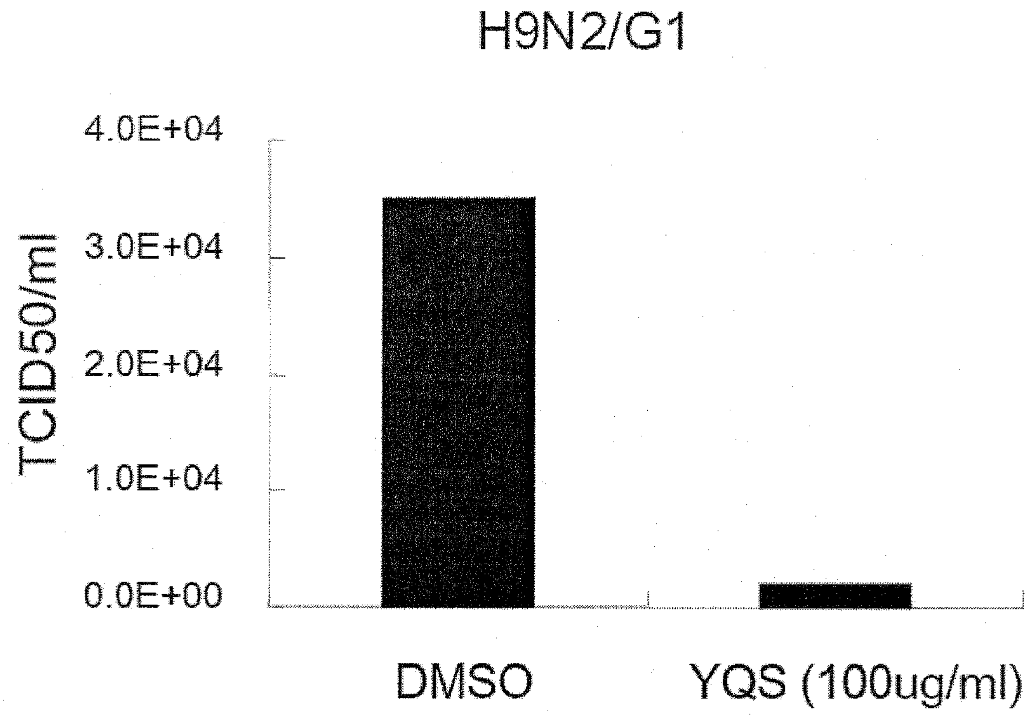


FIG. 13B

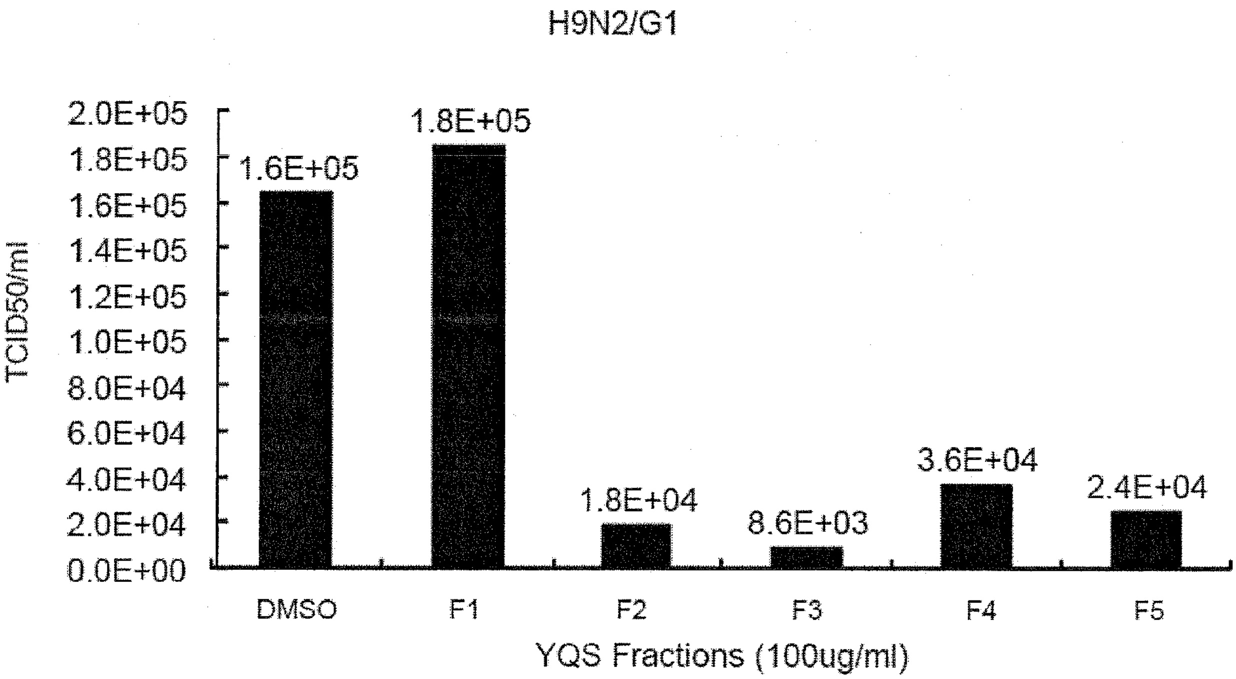


FIG. 13C

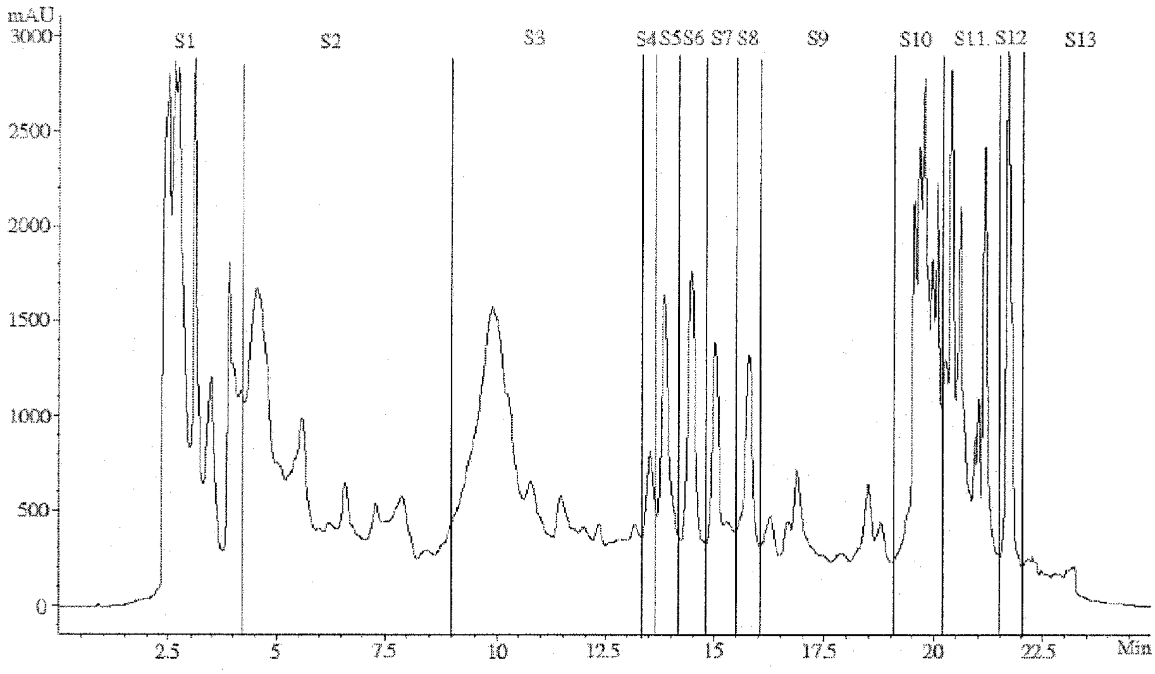


FIG. 14

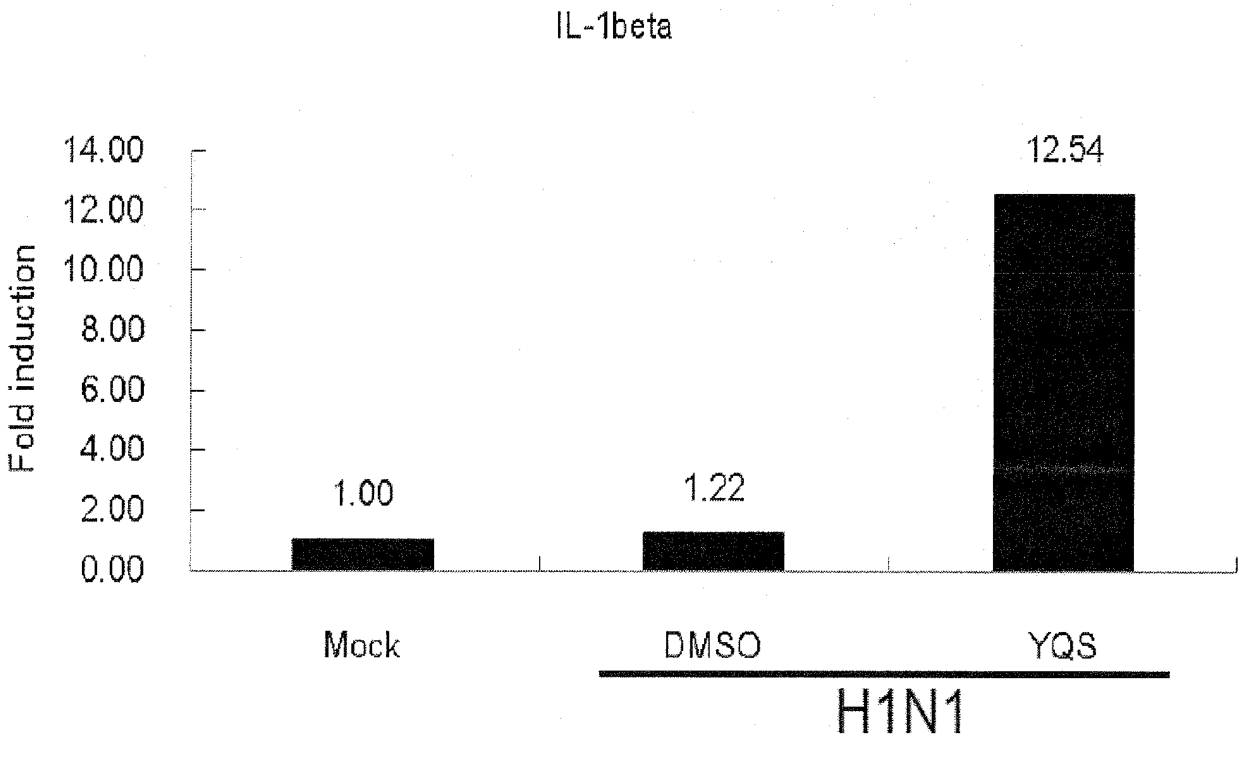


FIG. 15A

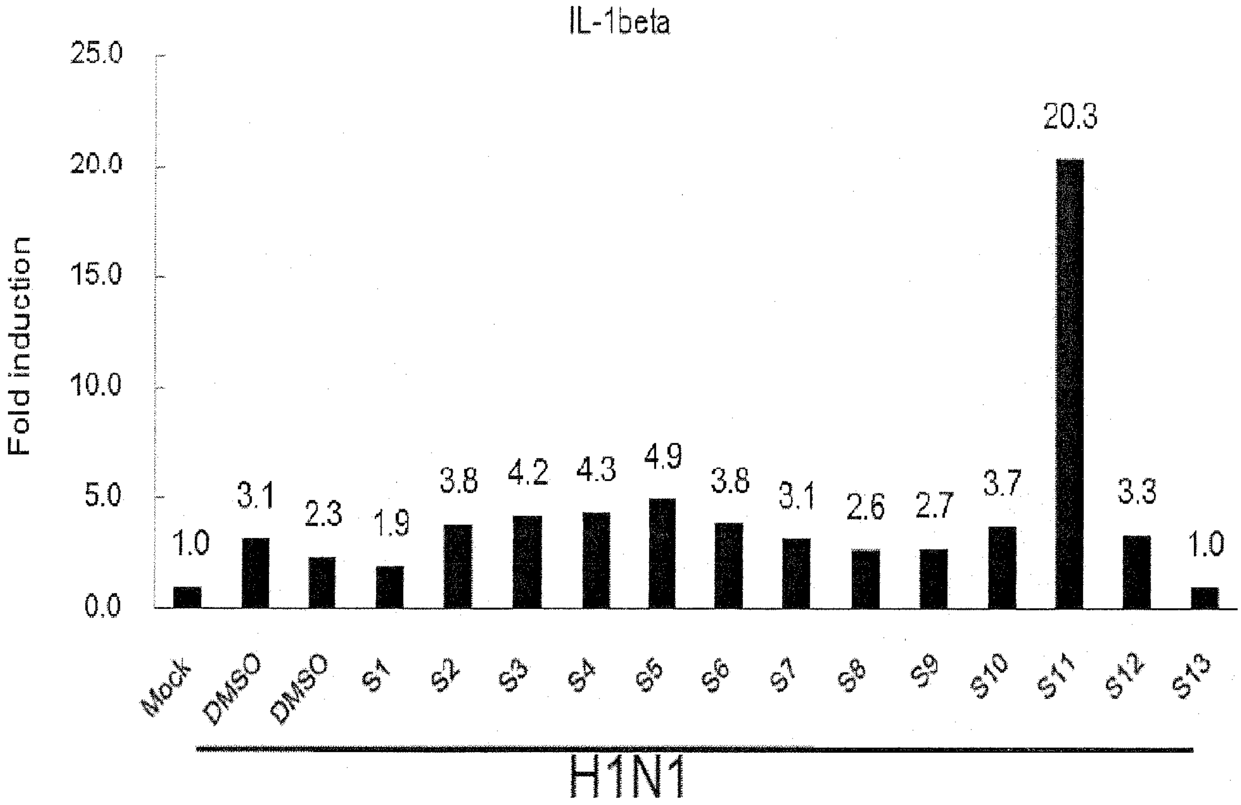
IL-1beta
Fold induction
25.0
20.0
15.0
10.0
5.0
0.0
1.0
3.1
2.3
1.9
3.8
4.2
4.3
4.9
3.8
3.1
2.6
2.7
3.7
20.3
3.3
1.0
Mock
DMSO
DMSO
S1
S2
S3
S4
S5
S6
S7
S8
S9
S10
S11
S12
S13
H1N1

FIG. 15B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 101390869 A
- US 6013632 A
- US 4078145 A
- US 6063383 A


### Non-patent literature cited in the description


- **MEIYING et al.** *Pharmacokinetics of Caffeic Acid in Rats, China Pharmacy,* 2008, vol. 19 (16), 1220
- Design of Prodrugs. Elsevier, 1985
- **FIELDS, B. et al.** Fields' Virology. Lippincott Williams and Wilkins, 2001
- The value of a database in surveillance and vaccine selection. **MACKEN, C. ; LU, H. ; GOODMAN, J. ; BOYKIN, L.** Options for the Control of Influenza IV. Elsevier Science, 2001, 103-106
- *Methods and Techniques in Virology,* 1993
- **NEUMANN G ; NODA T ; KAWAOKA Y.** Emergence and pandemic potential of swine-origin H1N1 influenza virus. *Nature,* 2009, vol. 459 (7249), 931-9
- **DAWOOD FS ; JAIN S ; FINELLI L ; SHAW MW ; LINDSTROM S ; GARTEN RJ ; GUBAREVA LV ; XU X ; BRIDGES CB ; UYEKI TM.** Emergence of a novel swine-origin influenza A (H1N1) virus in humans. *N Engl J Med.,* 2009, vol. 360 (25), 2605-15
- **AN J ; LEE DC ; LAW AH ; YANG CL ; POON LL ; LAU AS ; JONES SJ.** A novel small-molecule inhibitor of the avian influenza H5N1 virus determined through computational screening against the neuraminidase. *J Med Chem.,* 2009, vol. 52 (9), 2667-72
- **YANG CL ; CHIK SC ; LI JC ; CHEUNG BK ; LAU AS.** Identification of the bioactive constituent and its mechanisms of action in mediating the anti-inflammatory effects of black cohosh and related Cimicifuga species on human primary blood macrophages. *J Med Chem.,* 2009, vol. 52 (21), 6707-15
- **LEE DC ; YANG CL ; CHIK SC ; LI JC ; RONG JH ; CHAN GC ; LAU AS.** Bioactivity-guided identification and cell signaling technology to delineate the immunomodulatory effects of Panax ginseng on human promonocytic U937 cells. *J Transl Med.,* 2009, vol. 7, 34
- **JACKSON WT ; GIDDINGS TH, JR. ; TAYLOR MP ; MULINYAWE S ; RABINOVITCH M ; KOPITO RR ; KIRKEGAARD K.** Subversion of cellular autophagosomal machinery by RNA viruses. *PLoS Biol,* 2005, vol. 3, e156
- **M. CHIARA MAIUR ; EINAT ZALCKVAR ; ADI KIMCHI ; GUIDO KROEMER.** Self-eating and self-killing: crosstalk between autophagy and apoptosis. *Nat Rev Mol Cell Biol,* 2007, vol. 8 (9), 741-52
- **CHEUNG CY ; POON LL ; LAU AS ; LUK W ; LAU YL ; SHORTRIDGE KF ; GORDON S ; GUAN Y ; PEIRIS JS.** Induction of proinflammatory cytokines in human macrophages by influenza A (H5N1) viruses: a mechanism for the unusual severity of human disease?. *Lancet,* 2002, vol. 360, 1831-7
- **GUAN Y ; POON LL ; CHEUNG CY ; ELLIS TM ; LIM W ; LIPATOV AS ; CHAN KH ; STURM-RAMIREZ KM ; CHEUNG CL ; LEUNG YH.** H5N1 influenza: a protean pandemic threat. *Proc Natl Acad Sci U S A.,* 2004, vol. 101 (21), 8156-61
- **BEIGEL JH ; FARRAR J ; HAN AM ; HAYDEN FG ; HYER R ; DE JONG MD ; LOCHINDARAT S ; NGUYEN TK ; NGUYEN TH ; TRAN TH.** Writing Committee of the World Health Organization (WHO) Consultation on Human Influenza A/H5. (2005) Avian influenza A (H5N1) infection in humans. *N Engl J Med.,* vol. 353 (13), 1374-85
- **LEE DC ; CHEUNG CY ; LAW AH ; MOK CK ; PEIRIS M ; LAU AS.** p38 mitogen-activated protein kinase-dependent hyperinduction of tumor necrosis factor alpha expression in response to avian influenza virus H5N1. *J Virol,* 2005, vol. 79, 10147-54
- **HUI KP ; LEE SM ; CHEUNG CY ; NG IH ; POON LL ; GUAN Y ; IP NY ; LAU AS ; PEIRIS JS.** Induction of proinflammatory cytokines in primary human macrophages by influenza A virus (H5N1) is selectively regulated by IFN regulatory factor 3 and p38 MAPK. *J Immunol.,* 2009, vol. 182 (2), 1088-98

- **MOK CK ; LEE DC ; CHEUNG CY ; PEIRIS M ; LAU AS.** Differential onset of apoptosis in influenza A virus H5N1- and H1N1-infected human blood macrophages. *J Gen Virol.,* 2007, vol. 88, 1275-80
- Methods and Techniques in Virology. Marcel Dekker Inc, 1993, 32-33